(19) Europäisches Patentamt
European Patent Office
Office européen des brevets

(11) **EP 4 397 269 A1**

(12) **EUROPEAN PATENT APPLICATION**
published in accordance with Art. 153(4) EPC

(43) Date of publication:
**10.07.2024 Bulletin 2024/28**

(21) Application number: **22862836.8**

(22) Date of filing: **27.06.2022**

(51) International Patent Classification (IPC):
**A61B 34/20** (2016.01)

(52) Cooperative Patent Classification (CPC):
**A61B 34/20; A61B 5/06;** A61B 2034/2051;
A61B 2034/2053; A61B 2034/2072

(86) International application number:
**PCT/CN2022/101428**

(87) International publication number:
**WO 2023/029695 (09.03.2023 Gazette 2023/10)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **31.08.2021 CN 202111013350**

(71) Applicant: **Shanghai Microport Ep Medtech Co., Ltd.**
**Shanghai 201318 (CN)**

(72) Inventors:
• **WANG, Yanlei**
  **Shanghai 201318 (CN)**
• **WANG, Xinyi**
  **Shanghai 201318 (CN)**
• **SUN, Yiyong**
  **Shanghai 201318 (CN)**

(74) Representative: **Patentship**
**Patentanwaltsgesellschaft mbH**
**Paul-Gerhardt-Allee 50**
**81245 München (DE)**

(54) **POSITIONING SYSTEM, POSITIONING METHOD, INTERVENTIONAL SURGERY SYSTEM, ELECTRONIC DEVICE AND STORAGE MEDIUM**

(57)    A positioning system and method for an interventional device, an interventional surgical system, an electronic device and a storage medium. The positioning system includes a magnetic field generation unit (10), a stimulus control unit (20), a data collection unit (30) and a controller (40). A target region (101) is divided into a plurality of sub-regions (1011), and in a fitting phase, fitted models describing voltage-to-distance mappings in each sub-region (1011) are established with a learning instrument. In a subsequent positioning phase, distances between a third position on an applied instrument and electrode patches (102) are determined based on the fitted models for a sub-region (1011) where the applied instrument is situated, and a location of the third position is determined based on the distances between the third position and the electrode patches (102) and spatial position information of the electrode patches (102). The target region (101) is divided into the sub-regions (1011) so that each sub-region (1011) is small enough to allow an electric field to be distributed linearly therein and thus to allow the fitted models to be established for each sub-region (1011). Such fitted models enable more accurate positioning calculations.

Fig. 1

**Description**

**TECHNICAL FIELD**

The present invention relates to the field of medical devices and, in particular, to a positioning system and positioning method for an interventional device, an interventional surgical system, an electronic device and a storage medium.

**BACKGROUND**

In many medical scenarios, it is necessary to precisely determine the position of a medical device, such as catheter and implant, within a patient's body. To date, many positioning systems have been developed for medical devices. Typically, an energy field is created around a patient's body, such as a magnetic, electric or ultrasonic field, and a sensor capable of sensing the energy field is deployed on a device to be positioned, which is inserted into the body, such as a magnetic field sensor, an electric field sensor or an ultrasonic transducer. Energy field information sensed by the sensor is then converted into spatial position information. Magnetic field-based positioning systems are established and can provide high accuracy. However, magnetic field sensors used for this purpose are expensive and limited in use in large quantities. For this reason, in order to achieve sufficient positioning accuracy at controlled cost, a magnetic field-based positioning technique is usually used in combination with another positioning technique.

The following three conventional positioning methods are known.

In a first method, with a number of patch electrodes being placed on the surface of a patient's body, current stimuli are applied from an electrode deployed on a catheter inserted in the body, and impedances between the catheter electrode and the patches on the body surface are measured. At the same time, a magnetic field sensor deployed on the catheter provides accurate spatial position information. After a mapping is established between multiple positions and impedances measured at the positions, the catheter can be located and tracked based on impedance measurements of the ordinary electrodes.

In a second method, first and second coordinate systems are defined in electric and magnetic fields, respectively, and a mapping function is used to correlate the position of a magnetic field sensor, which is deployed on a catheter and in close proximity to an electrode, in the first coordinate system to its position in the second coordinate system. Later, based on the position of the magnetic field sensor in the first coordinate system and the mapping function, the position of the electrode can be adjusted and calibrated.

In a third method, three mutually orthogonal signals are applied to a region of interest of a patient's body, and a resulting voltage between an electrode on a catheter inserted in the body and a reference electrode is measured. Based on components of the voltage in the three orthogonal directions (X, Y, Z), coordinates of the position of the catheter electrode can be calculated.

However, these electric and magnetic field-based positioning methods are all insufficiently accurate, because an electric field will be nonlinearly distorted within a human body due to an uneven distribution of various substances therein. Such distortion creates challenges in accurately modeling an electric-field distribution within a human body using currently available linear models and accounts for the inaccuracy of the conventional positioning methods.

**SUMMARY OF THE INVENTION**

It is an object of the present invention to provide a positioning system and positioning method for an interventional device, an interventional surgical system, an electronic device and a storage medium, which can be used to position and track an interventional device within a target object with reduced computational burden and time consumption.

To this end, the present invention provides a positioning system for an interventional device comprising a learning instrument and an applied instrument, which are configured to be placed in a target region of a target object.

The positioning system comprises a magnetic field generation unit, a stimulus control unit, a data collection unit and a controller.

The magnetic field generation unit is configured to generate a magnetic field passing through the target object.

The stimulus control unit is configured to create an N-axis electric field by applying stimuli between at least three electrode patches disposed on a surface of the target object, where N≥3.

The controller is configured to divide the target region into a plurality of sub-regions.

The data collection unit is configured to: in a fitting phase, in each of the sub-regions, simultaneously collect magnetic field strength information at a first position on the learning instrument, magnetic field strength information at the electrode patches and voltage information at a second position on the learning instrument with respect to a reference position in all stimulus modes; and in a positioning phase, simultaneously collect magnetic field strength information at the electrode patches and voltage information at a third position on the applied instrument with respect to the reference position in all the stimulus modes.

The controller is further configured to: in the fitting phase, in each of the sub-regions, calculate spatial position information of the second position based on magnetic field strength information at the first position and on spatial distance information between the first and second positions and derive fitted models for the specific sub-region, which describe voltage-to-distance mappings, from voltage information at the second position with respect to the reference position in different stimulus modes and distance information between the second position and the electrode patches; and in the positioning phase, based on voltage information at the third position with respect to the reference position in the different stimulus modes and information about a sub-region where the applied instrument is situated, calculate spatial position information of the third position using the fitted models for the sub-region and spatial position information of the electrode patches, wherein the spatial position information of the electrode patches is calculated from magnetic field strength information at the electrode patches.

Optionally, the controller may be configured to configure the voltage information at the second position with respect to the reference position in the different stimulus modes and the distance information between the second position and the electrode patches into voltage-distance data pairs of a first type and derive the fitted models describing the voltage-to-distance mappings in the different stimulus modes in each of the sub-regions by fitting the voltage-distance data pairs.

Optionally, the controller may be configured to calculate distance information between the third position and at least three of the electrode patches using the fitted models in the different stimulus modes in the sub-region and calculate the spatial position information of the third position from the distance information between the third position and the at least three of the electrode patches and spatial position information of the at least three of the electrode patches.

Optionally, the controller may be configured to: in the fitting phase, for each of the electrode patches, gather voltage information at the second position with respect to the reference position in different stimulus modes associated with the electrode patch and distance information between the second position and the electrode patch into voltage-distance data pairs of a first type and derive fitted models in the different stimulus modes associated with the electrode patch, in each of the sub-regions; and

in the positioning phase, calculate distance information between the third position and the electrode patches using the fitted models for the electrode patches in a sub-region where the applied instrument is situated.

Optionally, the controller may be configured to select a fitted model satisfying a first predetermined criterion from the fitted models for the different stimulus modes associated with the electrode patch as a target fitted model and calculate distance information between the third position and the electrode patch using the target fitted model.

Optionally, the controller may be configured to select at least three electrode patches satisfying a second predetermined criterion from the electrode patches as target electrode patches, calculate distance information between the third position and the target electrode patches using the fitted models for a sub-region where the applied instrument is situated, and calculate spatial position information of the third position based on spatial position information of the target electrode patches.

Optionally, the applied instrument may further comprise a fourth position, wherein:

the data collection unit is further configured to collect magnetic field strength information at the fourth position in the positioning phase; and

the controller is further configured to, in the positioning phase, calculate spatial position and orientation information of the fourth position based on the magnetic field strength information at the fourth position and derive information about a sub-region wherein the applied instrument is situated from the spatial position and orientation information of the fourth position.

Optionally, the applied instrument may further comprise a fifth position, wherein:

the data collection unit is further configured to simultaneously collect voltage information at the fifth position with respect to the reference position in all the stimulus modes in the positioning phase; and

the controller is further configured to: in the positioning phase, calculate spatial position information of the fifth position based on spatial position and orientation information of the fourth position and spatial distance information between the fourth and fifth positions; calculate distance information between the fifth position and the electrode patches based on the spatial position information of the fifth position and spatial position information of the electrode patches; gather the voltage information at the fifth position with respect to the reference position in the different stimulus modes and the distance information between the fifth position and the electrode patches into voltage-distance data pairs of a second type; and update the fitted models for the sub-region using the voltage-distance data pairs of the second type.

Optionally, the learning and applied instruments may be provided as a single instrument, wherein the fourth position coincides with the first position, and the fifth position coincides with the second position.

Optionally, the controller may be further configured to: gather voltage information at the third position with respect to the reference position in different stimulus modes and distance information between the third position and the electrode patches into voltage-distance data pairs of a third type; screen out valid data pairs from the voltage-distance data pairs of the third type; and update the fitted models for the sub-region using the valid data pairs.

Optionally, the stimulus control unit may be configured to apply stimuli between the at least three electrode patches and fast switch between all the stimulus modes in a cyclic and continuous manner, wherein the data collection unit collects voltage information at the second and third positions with respect to the reference position in all the stimulus modes.

Alternatively, the stimulus control unit may be configured to simultaneously apply stimuli, each at a different frequency, between the at least three electrode patches, wherein the data collection unit collects and filters voltage information at the second and third positions with respect to the reference position, thereby obtaining voltage information at the second and third positions with respect to the reference position in all the stimulus modes.

Alternatively, the stimulus control unit may be configured to simultaneously apply stimuli, each at a different frequency, between the at least three electrode patches, wherein the data collection unit collects voltage information at the second and third positions with respect to the reference position, and the controller is configured to filter the voltage information at the second and third positions with respect to the reference position collected by the data collection unit, thereby obtaining voltage information at the second and third positions with respect to the reference position in all the stimulus modes.

Optionally, the positioning system may further comprise a communication control unit, which is configured to connect the controller to the magnetic field generation unit, the stimulus control unit and the data collection unit, thereby controlling communication and data transmission between the controller and the magnetic field generation unit, the stimulus control unit and the data collection unit.

Optionally, the positioning system may further comprise a display unit, which is communicatively connected to the controller and configured to display a position, orientation, shape and/or travel path of the learning instrument and/or the applied instrument in the target object.

Optionally, a first magnetic field sensor may be disposed at the first position, and second magnetic field sensors may be disposed on the electrode patches, wherein the data collection unit collects magnetic field strength information at the first position through the first magnetic field sensor and collects magnetic field strength information at the electrode patches through the second magnetic field sensors.

Additionally, a first voltage sensor may be disposed at the second position, and a second voltage sensor may be disposed at the third position, wherein the data collection unit collects voltage information at the second position with respect to the reference position through the first voltage sensor and collects voltage information at the third position with respect to the reference position through the second voltage sensor.

To the above end, the present invention further provides an interventional surgical system comprising the positioning system and the interventional device as defined above.

To the above end, the present invention further provides a positioning method for an interventional device comprising a learning instrument and an applied instrument, which are configured to be placed in a target region of a target object. The positioning method comprises:

> dividing the target region into a plurality of sub-regions;
> in a fitting phase, obtaining magnetic field strength information at a first position of the learning instrument, magnetic field strength information at at least three electrode patches disposed on a surface of the target object and voltage information at a second position on the learning instrument with respect to a reference position in all stimulus modes, which are simultaneously collected in each of the sub-regions, calculating spatial position information of the second position based on the magnetic field strength information at the first position and on spatial distance information between the first and second positions, and deriving fitted models describing voltage-to-distance mappings in each of the sub-regions from voltage information at the second position with respect to the reference position at different stimulus modes and from distance information between the second position and the electrode patches; and
> in a positioning phase, obtaining voltage information simultaneously collected in different stimulus modes at a third position on the applied instrument with respect to the reference position, information about a sub-region where the applied instrument is situated and spatial position information of the electrode patches and calculating spatial position information of the third position using the fitted models for the sub-region and spatial position information of the electrode patches, wherein the spatial position information of the electrode patches is calculated from magnetic field strength information at the electrode patches.

Optionally, deriving the fitted models describing the voltage-to-distance mappings in each of the sub-regions from the voltage information at the second position with respect to the reference position at the different stimulus modes and from the distance information between the second position and the electrode patches may comprise:
gathering the voltage information at the second position with respect to the reference position in the different stimulus modes and the distance information between the second position and the electrode patches into voltage-distance data pairs of a first type and deriving the fitted models describing the voltage-to-distance mappings in the different stimulus modes in each of the sub-regions by fitting the voltage-distance data pairs of the first type.

Optionally, deriving the fitted models describing the voltage-to-distance mappings in each of the sub-regions from the voltage information at the second position with respect to the reference position at the different stimulus modes and from the distance information between the second position and the electrode patches may comprise:

for each of the electrode patches, gathering voltage information at the second position with respect to the reference position in different stimulus modes associated with the electrode patch and distance information between the second position and the electrode patch into voltage-distance data pairs of a first type and derive fitted models in the different stimulus modes associated with the electrode patch in each of the sub-regions.

Optionally, calculating the spatial position information of the third position using the fitted models for the sub-region and the spatial position information of the electrode patches may comprise:

calculating distance information between the third position and the electrode patches using the fitted models for the electrode patches in the sub-region; and
calculating the spatial position information of the third position based on the distance information between the third position and the electrode patches and spatial position information of the electrode patches.

Optionally, calculating the distance information between the third position and the electrode patches using the fitted models for the electrode patches in the sub-region may comprise:

selecting a fitted model satisfying a first predetermined criterion from the fitted models for the different stimulus modes associated with the electrode patch as a target fitted model; and
calculating the distance information between the third position and the electrode patch using the target fitted model.

Optionally, calculating the spatial position information of the third position using the fitted models for the sub-region and the spatial position information of the electrode patches may comprise:

selecting at least three electrode patches satisfying a second predetermined criterion from the electrode patches as target electrode patches; and
calculating distance information between the third position and the target electrode patches using the fitted models for the sub-region and calculating the spatial position information of the third position based on spatial position information of the target electrode patches.

Optionally, the applied instrument may further comprise a fourth position, wherein the positioning method further comprises:
in the positioning phase, collecting magnetic field strength information at the fourth position, calculating spatial position and orientation information of the fourth position based on the magnetic field strength information at the fourth position and deriving information about a sub-region wherein the applied instrument is situated from the spatial position and orientation information of the fourth position.

Optionally, the applied instrument may further comprise a fifth position, wherein the positioning method further comprises:
in the positioning phase, simultaneously collecting voltage information at the fifth position with respect to the reference position in all the stimulus modes, calculating spatial position information of the fifth position based on spatial position and orientation information of the fourth position and spatial distance information between the fourth and fifth positions, calculating distance information between the fifth position and the electrode patches based on the spatial position information of the fifth position and spatial position information of the electrode patches, gathering the voltage information at the fifth position with respect to the reference position in the different stimulus modes and the distance information between the fifth position and the electrode patches into voltage-distance data pairs of a second type, and updating the fitted models for the sub-region using the voltage-distance data pairs of the second type.

Optionally, the positioning method may further comprise:
gathering voltage information at the third position with respect to the reference position in different stimulus modes and distance information between the third position and the electrode patches into voltage-distance data pairs of a third type; screening out valid data pairs from the voltage-distance data pairs of the third type; and updating the fitted models for the sub-region using the valid data pairs.

To the above end, the present invention further provides an electronic device including a processor and a memory device. The memory device stores therein a computer program, which, when executed by the processor, implements the positioning method as defined above.

To the above end, the present invention further provides a readable storage medium storing therein a computer program, which, when executed by a processor, implements the positioning method as defined above.

Compared with the prior art, the positioning system and positioning method, interventional surgical system, electronic device and storage medium have the advantages as follows: a target region is divided into a plurality of sub-regions, and in a fitting phase, fitted models describing voltage-to-distance mappings in each sub-region are established with a learning instrument. In a subsequent positioning phase, distances between a third position on an applied instrument and electrode patches are determined based on the fitted models for a sub-region where the applied instrument is situated, and a position of the third position is determined based on the distances between the third position and the electrode patches and spatial position information of the electrode patches. In this way, the applied instrument can be positioned, and position, orientation and shape information of the applied instrument in the target region of the target object can be determined. According to the present invention, the target region is divided into the sub-regions so that each sub-region is small enough to allow an electric field to be distributed linearly therein and thus to allow the fitted models to be established for each sub-region. Using such fitted models, the distances between the third position and the electrode patches can be more accurately determined, achieving more accurate positioning calculations. Further, since fitted models are established for each sub-region according to the present invention, computational burden in the fitting calculations is small. Thus, effective reductions in computational burden and time savings can be achieved.

## BRIEF DESCRIPTION OF THE DRAWINGS

Fig. 1 schematically illustrates modules required for operation of a positioning system for an interventional device according to an embodiment of the present invention.
Fig. 2 is a schematic flowchart of a process of using the positioning system of Fig. 1.
Fig. 3 is a schematic flowchart of a positioning algorithm according to an embodiment of the present invention.
Fig. 4a is a schematic diagram showing the structure of a loop catheter according to an embodiment of the present invention.
Fig. 4b schematically illustrates an arrangement of sensors (positions) on the loop catheter of Fig. 4a.
Fig. 4c schematically illustrates a position result for the loop catheter of Fig. 4a at a certain instant of time.
Fig. 5 schematically illustrates how a target region is divided according to an embodiment of the present invention.
Figs. 6a to 6e show schematic scatter plots of voltage-distance data pairs accumulated in different stimulus modes for a sub-region during a fitting phase.
Fig. 7 schematically illustrates distances from a second position to electrode patches according to an embodiment of the present invention.
Fig. 8 is a schematic block diagram of an electronic device according to an embodiment of the present invention.

List of Reference Numerals

100 - target object; 101 - target region; 1011 - sub-region; 102, 102H, 102F, 102C, 102B, 102R, 102L - electrode patch; 103P - first magnetic field sensor; 103Q - second magnetic field sensor; 104 - electrode; 104S, 104S1, 104S2 - first electrode; 104T, 104T1, 104T2 - second electrode; 105 - interventional device; 106 - manipulating handle; 107 - magnetic field; 108 - display unit; 109 - three-dimensional body cavity model; 110 - tip portion model; 111 - travel path;

10 - magnetic field generation unit; 20 - stimulus control unit; 30 - data collection unit; 40 - controller; 50 - communication control unit;

301 - processor; 302 - communication interface; 303 - memory device; 304 - communication bus.

## DETAILED DESCRIPTION

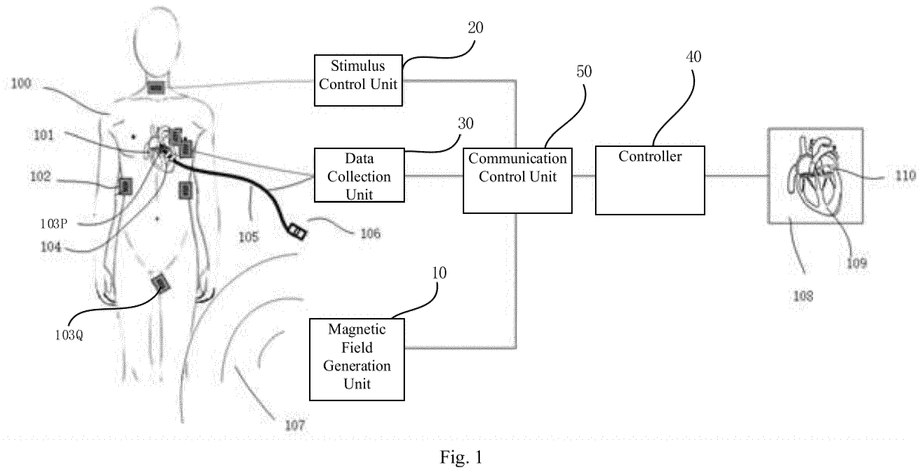

Positioning system and positioning methods for an interventional device, interventional surgical systems, electronic devices and storage media proposed in the present invention will be described in greater detail below with reference to Figs. 1 to 8 and to specific embodiments thereof. From the following description, advantages and features of the present invention will become more apparent. It is to be noted that the figures are provided in a very simplified form not necessarily drawn to exact scale and for the only purpose of facilitating easy and clear description of the embodiments.

In order that the objects, features and advantages of the present invention can be more apparent and readily understood, reference is to be made to the accompanying drawings. It would be recognized that architectural, proportional, dimensional and other details in the figures are presented only for the purpose of facilitating, in conjunction with the disclosure herein, the understanding and reading of those familiar with the art, rather than being intended to limit conditions under which the present invention can be implemented. Therefore, any and all architectural modifications, proportional variations or dimensional changes that achieve the same or similar benefits and objects as the present invention are considered to fall within the scope of the teachings herein.

It is to be noted that, as used herein, relational terms such as first and second, etc., are only used to distinguish one entity or operation from another entity or operation, and do not necessarily require or imply these entities having such an order or sequence. Moreover, the terms "comprise," "include," or any other variations thereof are intended to cover a non-exclusive inclusion within a process, method, article, or apparatus that comprises a list of elements including not only those elements but also those that are not explicitly listed, or other elements that are inherent to such processes, methods, goods, or equipment. In the case of no more limitation, the element defined by the sentence "includes a ..." does not exclude the existence of another identical element in the process, the method, or the device including the element.

In principle, the present invention seeks to provide a positioning system and positioning method for an interventional device, an interventional surgical system, an electronic device and a storage medium, which can be used to locate and track an interventional device within a target object with reduced computational burden and time consumption.

It is to be noted that electronic devices according to embodiments of the present invention may be personal computers, mobile terminals or the like. The mobile terminals may be hardware devices running various operating systems (OS), such as mobile phones and tablet computers.

To achieve the above goal, the present invention provides a positioning system for an interventional device, which includes a learning instrument and an applied instrument, both configured to be placed in a target region of a target object. The positioning system includes a magnetic field generation unit, a stimulus control unit, a data collection unit and a controller.

The magnetic field generation unit is configured to generate a magnetic field passing through the target object.

The stimulus control unit is configured to apply stimuli between at least three electrode patches disposed on a surface of the target object and thereby generate an N-axis electric field within the target object, where $N \geq 3$.

The controller is configured to divide the target region into a plurality of sub-regions.

The data collection unit is configured to: in a fitting phase, in each of the sub-regions, simultaneously collect magnetic field strength information at a first position on the learning instrument, magnetic field strength information at the electrode patches and voltage information at a second position on the learning instrument with respect to a reference position in all stimulus modes; and in a positioning phase, simultaneously collect magnetic field strength information at the electrode patches and voltage information at a third position on the applied instrument with respect to the reference position in all the stimulus modes.

The controller is further configured to: in the fitting phase, in each of the sub-regions, calculate spatial position information of the second position based on magnetic field strength information at the first position and on spatial distance information between the first and second positions and derive fitted models for the specific sub-region, which describe voltage-to-distance mappings, from voltage information at the second position with respect to the reference position in different stimulus modes and distance information between the second position and the electrode patches; and in the positioning phase, based on voltage information at the third position with respect to the reference position in the different stimulus modes and information about a sub-region where the applied instrument is situated, calculate spatial position information of the third position using the fitted models for the sub-region and spatial position information of the electrode patches, wherein the spatial position information of the electrode patches is calculated from magnetic field strength information at the electrode patches.

Thus, operation of the positioning system of the present invention is divided into a fitting phase and a positioning phase, and a target region is divided into a plurality of sub-regions. In the fitting phase, fitted models describing voltage-to-distance mappings in each sub-region are established with a learning instrument. In the subsequent positioning phase, distances between a third position on an applied instrument and electrode patches are determined based on the fitted models for a sub-region where the applied instrument is situated, and a position of the third position is determined based on the distances between the third position and the electrode patches and spatial position information of the electrode patches. In this way, the applied instrument can be located, and position, orientation and shape information of the applied instrument in the target region of the target object can be determined. According to the present invention, the target region is divided into the sub-regions so that each sub-region is small enough to allow an electric field to be distributed linearly therein and thus to allow the fitted models to be established for each sub-region. Using such fitted models, the distances between the third position and the electrode patches can be more accurately determined, achieving more accurate positioning calculations. Further, according to the present invention, since fitted models are established for each sub-region, computational burden in the fitting calculations is small. Thus, effective reductions in computational burden and time savings can be achieved. It is to be noted that, as would be appreciated by those skilled in the art, spatial position and orientation information of the first position may be first calculated from the magnetic field strength information at the first position, and the spatial position information of the second position may be then derived from the spatial position and orientation information of the first position and the spatial distance information between the first and second positions. Moreover, the distance information between the second position and the electrode patches may be calculated from the spatial position information of the second position and the spatial position information of the electrode patches.

Further, the controller may be configured to gather the voltage information at the second position with respect to the reference position in the different stimulus modes and the distance information between the second position and the electrode patches into voltage-distance data pairs of a first type and derive the fitted models describing the voltage-to-distance mappings in the different stimulus modes in each of the sub-regions by fitting the voltage-distance data pairs. Thus, through gathering the voltage information at the second position with respect to the reference position in different stimulus modes and the distance information between the second position and the electrode patches into voltage-distance data pairs of the first type and fitting the voltage-distance data pairs, the fitted models in the different stimulus modes in each sub-region can be obtained in a fast and accurate way.

Further, the controller may be configured to calculate distance information between the third position and at least three of the electrode patches using the fitted models in the different stimulus modes in the sub-region and calculate the spatial position information of the third position from the distance information between the third position and the at least three of the electrode patches and spatial position information of the at least three of the electrode patches. Thus, according to the present invention, the spatial position information of the third position can be calculated from the distance information between the third position and the at least three of the electrode patches and spatial position information of the at least three of the electrode patches. This allows the spatial position information of the third position to be calculated more accurately.

A positioning system for an interventional device according to the present invention is described in detail below with reference to Figs. 1 to 5.

Fig. 1 schematically illustrates modules required for operation of a positioning system for an interventional device according to an embodiment of the present invention. As shown in Fig. 1, the positioning system of the present invention is used on a target object 100, which may be a human, an animal or another suitable conductive object. A tip portion of the interventional device 105 is inserted into a target region 101 of the target object 100, which may be a cardiac chamber, for example.

The interventional device 105 may be any of various medical catheters, such as electrophysiology catheters and multi-arm and loop catheters used for ablation or mapping, and other implantable devices. When the interventional device 105 is an electrophysiology catheter, it is generally used for diagnosis or treatment, e.g., for electrophysiological mapping, delivery of ablation energy, etc. In the embodiment shown in Fig. 1, the interventional device 105 is an electrophysiology catheter, which is usually advanced into the target region 101 (e.g., a cardiac chamber) through a vascular access. The tip portion of the interventional device 105 may be either fixed or steerable. A portion of the interventional device 105 left outside the target object usually includes a manipulating handle 106, and a physician can manipulate the manipulating handle 106 to control the shape or orientation of the tip portion of the interventional device 105.

The interventional device 105 is provided thereon with two types of sensors: a magnetic field sensor (for the sake of distinction, this magnetic field sensor arranged on the interventional device is referred to hereinafter as the "first magnetic field sensor 103P") and voltage sensors (which are ordinary electrodes, referred to hereinafter as the "electrodes 104"). Specifically, the first magnetic field sensor 103P is disposed at a first position of the interventional device, a first voltage sensor (referred to hereinafter briefly as the "first electrode 104S") at a second position, and a second voltage sensor (referred to hereinafter briefly as the "second electrode 104T") at a third position. Signals sensed by the first magnetic field sensor 103P and the electrodes 104 are transmitted through wires inside the interventional device 105 to a data collection unit 30. There may be one or more of each of the first magnetic field sensor 103P, the first voltage sensor and the second voltage sensor.

Depending on the functionality provided by the interventional device 105 during positioning, it may include a learning instrument and an applied instrument, both required to be provided thereon with at least one first magnetic field sensor 103P (information about the sub-region where the applied instrument is during a positioning phase is determined using the first magnetic field sensor 103P provided thereon). The learning instrument is further provided thereon with at least one first electrode 104S disposed at a known fixed spacing from the first magnetic field sensor 103P, and the applied instrument is further provided thereon with a number of second electrodes 104T to be positioned. Of course, the terms "learning instrument" and "applied instrument" are only intended to distinguish different instrument functions, and in practical use, a single instrument may function as both the learning and applied instruments. For example, in the case of the interventional device 105 being implemented as a medical catheter, it may include two catheters functioning respectively as a learning catheter (i.e., the learning instrument) and an applied catheter (i.e., the applied instrument). Alternatively, it may include only one catheter functioning as both a learning catheter and an applied catheter. Of course, in further embodiments, the interventional device may also include at least three catheters, in which one or more catheters function as learning catheters and the remaining one(s) as applied catheter(s). All these embodiments are contemplated, and the present invention is not limited in this regard.

The following description is set forth in the exemplary context of the interventional device 105 being implemented as a medical catheter. In the description below, it is assumed that the medical catheter is provided with one first magnetic field sensor 103P, one first electrode 104S disposed at a known fixed spacing from the first magnetic field sensor 103P and a number of second electrodes 104T to be positioned, and function as both a learning catheter and an applied catheter. The spacing is a Euclidean distance in a three-dimensional space. In this way, position information of the first electrode 104S can be calculated based on spatial position and orientation information of the first magnetic field sensor 103P, while position information of the second electrodes 104T can be determined according to a positioning method described herein. It is to be noted that, in the context of the present invention, the first position of the medical catheter is where the first magnetic field sensor 103P is located, the second position is where the first electrode 104S is located, and the third positions are where the respective second electrodes 104T are located. Accordingly, calculating spatial positions of the second and third positions is equivalent to calculating spatial positions of the first electrode 104S and the second electrodes 104T.

The magnetic field sensor typically includes a number of coils capable of sensing the strength of a magnetic field at their positions, which is generated by a magnetic field generation unit 10. Based on magnetic field strength information sensed by the magnetic field sensor, a controller 40 can calculate spatial position and orientation information of the magnetic field sensor. This calculation may be accomplished with a conventional magnetic positioning technique, which would be well known to those of ordinary skill in the art and, therefore, need not be described in further detail herein. The "spatial position information" generally refers to X, Y and Z coordinates in a three-dimensional Cartesian coordinate system, and the "orientation information" refers to an orientation vector and, more generally, a unit orientation vector of the magnetic field sensor.

Each of the electrodes 104 (i.e., voltage sensors) is made of a metal with good biocompatibility, such as a platinum-iridium alloy or gold, and is used to measure a voltage at its position with respect to a given reference position. The reference position (not depicted in Fig. 1) is generally a relatively constant position and may be selected as a surface or internal position of the target object 100 (e.g., the coronary sinus). Preferably, a reference electrode is placed at the reference position (e.g., the coronary sinus). In addition to measuring the voltage, each electrode 104 may also be used to measure an electrophysiological signal or release ablation energy.

The magnetic field generation unit 10 is configured to generate a magnetic field 107 and is typically fixed near the target object 100 so that the generated magnetic field 107 can pass through the target object 100. The magnetic field 107 can be sensed by the magnetic field sensor, allowing the spatial position and orientation of the magnetic field sensor to be determined.

Typically, three or more electrodes, which are usually sheet-shaped, are attached to the surface of the target object 100 (referred to hereinafter as the "electrode patches 102" in order to distinguish them from the aforementioned electrodes 104 deployed on the medical catheter). With each pair of the electrode patches 102, a stimulus control unit 20 can apply a particular electric field to a human body as a stimulus, which is preferred to be a constant-current or constant-voltage signal. Preferably, the applied current flows through the target region 101. The stimulus control unit 20 applies stimuli through multiple pairs of the electrode patches 102, and the data collection unit 30 collects voltages of each of the electrodes 104 on the medical catheter (including 104S and 104T) with respect to the reference position in all stimulus modes at the same sampling time, which are used in subsequent positioning calculations. In addition, each electrode patch 102 may be provided with a magnetic field sensor (for the sake of distinction, the magnetic field sensors on the electrode patches 102 are referred to hereinafter as the "second magnetic field sensors 103Q") configured to provide spatial position information. Accordingly, for each electrode patch 102, the controller may calculate spatial position information of the second magnetic field sensor 103Q thereon based on magnetic field strength information sensed by the second magnetic field sensor 103Q and take the spatial position information of the second magnetic field sensor 103Q as spatial position information of the electrode patch 102. Here, the "same sampling time" refers to an instant of time at which magnetic field strength data from the magnetic field sensors (including the first magnetic field sensor 103P and the second magnetic field sensors 103Q) and the voltage information of each electrode 104 with respect to the reference position are collected. That is, the data collection unit 30 collects the magnetic field strength data and the voltage information at the same sampling time. The "stimulus mode" refers to modes of currents each created by a respective stimulus applied by the stimulus control unit 20 through two (one pair) of the electrode patches 102, also called stimulus axes. When there are N stimulus axes, it can be considered that the stimulus control unit 20 applies stimuli through N pairs of electrode patches 102, which create an N-axis electric field in the target object 100. Generally, the electrode patches 102 are maintained at fixed positions on the body surface throughout the surgical procedure.

In order to obtain voltage information of the second and third positions (i.e., each electrode 104) with respect to the reference position in all stimulus modes at the same sampling time, any of the following schemes may be employed. The stimulus control unit 20 may apply stimuli between the at least three electrode patches 102 and fast switch between all stimulus modes in a cyclic and continuous manner, and the data collection unit 30 may collect the voltage information of the second and third positions (i.e., each electrode 104) with respect to the reference position in all the stimulus modes.

Alternatively, the stimulus control unit 20 may simultaneously apply stimuli, each at a different frequency, between the at least three electrode patches 102, and the data collection unit 30 may collect voltage information of the second and third positions (i.e., each electrode 104) with respect to the reference position, thereby obtaining the voltage information of the second and third positions (i.e., each electrode 104) with respect to the reference position in all stimulus modes.

Still alternatively, the stimulus control unit 20 may simultaneously apply stimuli, each at a different frequency, between the at least three electrode patches 102, and the data collection unit 30 may collect voltage information of the second and third positions (i.e., each electrode 104) with respect to the reference position. The controller 40 may filter the voltage information of the second and third positions (i.e., each electrode 104) with respect to the reference position collected by the data collection unit 30, thereby obtaining the voltage information of the second and third positions (i.e., each electrode 104) with respect to the reference position in all stimulus modes.

In the embodiment shown in Fig. 1, there are six electrode patches 102, deployed respectively at the nape of the neck (H), groin (F), chest (C), back (B), left axilla (L) and right axilla (R). The stimulus control unit 20 may apply a stimulus through each chosen pair of electrode patches 102. Thus, there are totally 15 possible stimulus modes or axes: RL, HF, CB, CR, CL, CH, CF, RB, LB, HB, FB, RF, LF, HR and HL. It is to be noted that the present invention is not limited to applying a stimulus between each two of the electrode patches 102 by the stimulus control unit 20, any suitable stimulus application scheme is possible, as long as an N-axis (N>_3) electric field is produced in the target region 101 of the target object 100 as a result of stimuli being applied between N pairs of electrode patches 102. In practical use, it is possible that a current or currents through the target region 101 resulting from a stimulus or stimuli applied along one or more stimulus axes is/are too small to allow corresponding voltage(s) to be detected by the electrodes 104 in the target region 101, or to have amplitude much smaller than that of its/their counterparts. In this situation, the stimulus control unit 20 may give up the one or more stimulus modes (invalid stimulus modes) in the subsequent calculations. It is within the scope of the present invention that, when one or more stimulus modes are discarded, stimulus modes involved in the calculations include n ($\geq$3) valid stimulus modes selected from all the possible stimulus modes. Both N and n are natural numbers, and when all the stimulus modes are valid, they may be equal to each other. Of course, if such screening is omitted, then the stimulus modes involved in the calculations may include all the stimulus modes.

In order to obtain voltage information of each electrode 104 with respect to the reference position in all stimulus modes at the same sampling time, either of the following time-multiplexing and frequency-multiplexing schemes may be employed. The time-multiplexing scheme involves fast switching between the 15 stimulus modes in a cyclic and continuous manner and collecting voltage information of each electrode 104 with respect to the reference position in all stimulus modes. When the switching occurs at a sufficiently high frequency, it can be considered that the voltage information of each electrode 104 with respect to the reference position is collected in all stimulus modes at the same time. The switching frequency may be 200 kHz or 300 kHz. The frequency-multiplexing scheme involves applying signals along the 15 stimulus axes at different frequencies and filtering signals collected from the electrodes 104 by the data collection unit 30 or the controller 40, thereby obtaining voltages in all the stimulus modes. For example, constant-current or constant-voltage signals may be applied at 10 kHz, 10.5 kHz, 11 kHz, 11.5 kHz and so forth. The frequency-multiplexing scheme is preferred.

The data collection unit 30 is configured to process data from all the sensors in the positioning system, including the magnetic field sensors and voltage sensors (i.e., the electrodes 104). This typically involves amplification, filtering and analog-to-digital conversion. In the context of the positioning functionality according to the present invention, the data collection unit 30 is required to collect the following data in each sampling time:

1. magnetic field strength data of the magnetic field sensors (including the first magnetic field sensor 103P and the second magnetic field sensors 103Q); and
2. voltage data of the electrodes 104 (including the first electrode 104S and second electrodes 104T) with respect to the reference position in all stimulus modes.

As shown in Fig. 1, the positioning system further includes a communication control unit 50, which is configured to connect the controller 40 to the stimulus control unit 20, the data collection unit 30 and the magnetic field generation unit 10 and control communication and data transmission between the controller 40 and the magnetic field generation unit 10, stimulus control unit 20 and data collection unit 30. The communication control unit 50 serves as a "relay" for signal and data transmission, which may be wired transmission via serial, SPI (Serial Peripheral Interface), I$^2$C, network, USB or other interfaces, or wireless transmission, such as WiFi or Bluetooth transmission. In addition to the above-described various units and modules in the positioning system, the communication control unit 50 may also be connected to other devices, such as those commonly used in surgical procedures, such as RF ablation, CT, MRI and ECG systems.

The controller 40 is typically a general-purpose computer and serves as a core component for system control and data processing. It can control operation of the system through the communication control unit 50. Specifically, the controller 40 is configured to divide the target region into a plurality of sub-regions. Apart from this, the controller 40 may provide the functions of:

1. activating or deactivating the magnetic field generation unit 10 and causing the stimulus control unit 20 to operate in a desired mode;
2. causing the data collection unit 30 to simultaneously collect magnetic field strength of the magnetic field sensors (including the first magnetic field sensor 103P and the second magnetic field sensors 103Q) and voltages of the electrodes 104 (including the first electrode 104S and the second electrodes 104T) with respect to the reference position;
3. creating fitted models describing voltage-to-distance mappings for the sub-regions, based on voltages at the first electrode 104S and distances between the first electrode 104S and the electrode patches 102; and
4. based on the fitted models for the sub-regions and voltages at the second electrodes 104T, calculating distances between the second electrodes 104T and the electrode patches 102 and hence spatial positions of the second electrodes 104T.

The controller 40 generally performs these tasks under the control of a software program, which may be downloaded to a computer or accessed via a network. The controller 40 may be provided with a memory device for storing data from the sensors or relevant data produced during positioning.

Preferably, as shown in Fig. 1, the positioning system further includes a display unit 108, which is typically a display device, such as CRT or LCD. The display unit 108 is communicatively connected to the controller 40 and configured to display the position, orientation, shape and/or travel path of the applied instrument of the interventional device 105 in the target object 100, which are determined by the controller 40 according to a positioning result for the third positions. It would be appreciated that a three-dimensional model of the target object 100 may be pre-created before the surgical procedure begins. Accordingly, after the spatial positions of the third positions are determined, the applied catheter may be modeled based on the spatial positions of the third positions, and the position, orientation and shape of the applied catheter in the target object 100 may be determined based on the model. Through determining the position, orientation and shape of the applied catheter in the target object 100 at each sampling time, its travel path in the target object 100 can also be determined. In the embodiment shown in Fig. 1, the display unit 108 displays a three-dimensional model 109 of a body cavity and a model 110 of the tip portion of the applied catheter in the body cavity rendered based on a positioning result of the controller 40. Of course, in other embodiments, the display unit 108 may also be configured to display the position, orientation, shape and/or the travel path of the learning instrument in the target object 100, which are determined by the controller 40 according to a positioning result for the first and second positions.

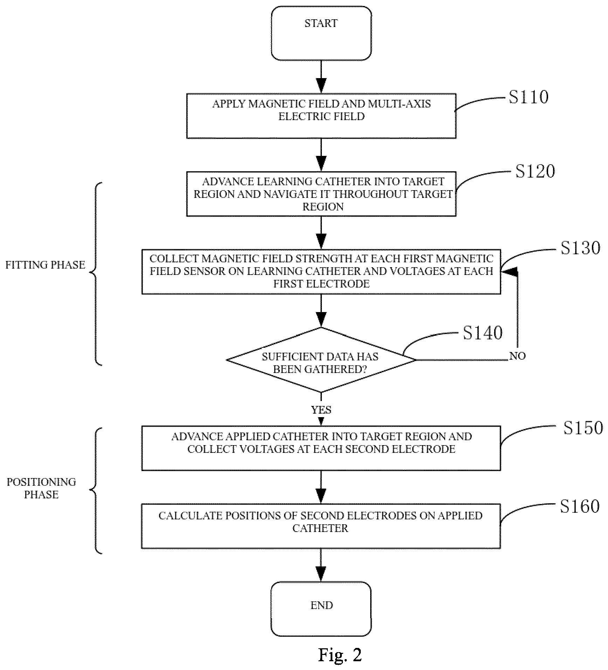

Reference is now made to Fig. 2, a schematic flowchart of a process of using the positioning system of the present invention. As shown in Fig. 2, the process includes the steps as follows.

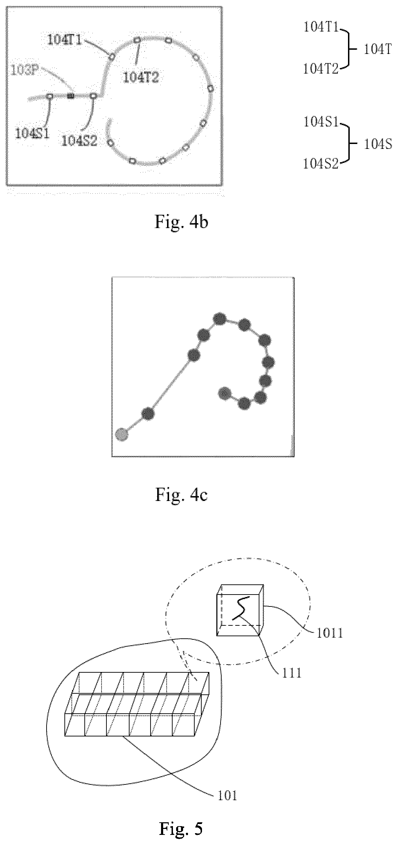

Step S 110: At the beginning of a surgical procedure, apply a magnetic field and a multi-axis electric field to a human body and divide a target region 101 into a plurality of sub-regions 1011 (see Fig. 5). Generally, the magnetic field is applied by the magnetic field generation unit 10, and the multi-axis electric field results from current stimuli applied between respective pairs of a plurality of electrode patches 102 attached to the surface of the target object 100. The stimuli may be either constant-current stimuli or constant-voltage stimuli. The target region 101 is divided into a plurality of sub-regions 1011 so that, despite an overall non-linear distribution of the electric field within the object, its distribution in a small region is almost linear. It is just such linearity of the electric field in each small sub-region that allows a linear voltage versus distance relationship to exist therein, which serves as a basis for meaningful linear fitting calculations in relation to the sub-region.

As detailed below, the process is divided into two phases: a fitting phase and a positioning phase. The learning catheter is operated in the fitting phase, and the applied catheter is operated in the positioning phase. The terms "learning catheter" and "applied catheter" are only intended to distinguish different catheter functions, and in fact, a single catheter may be used to accomplish the operation in both the fitting and positioning phases. The fitting phase begins earlier than the positioning phase, but there may be a time overlap between the two phases. The fitting phase may begin as soon as the learning catheter is advanced into the target region 101 (e.g., a cardiac chamber) through a vascular access. At least one first magnetic field sensor 103P and at least one first electrode 104S are integrated on the learning catheter at a fixed spacing therebetween. Information of this spacing is known, allowing position information of the first electrode 104S to be derived from position and orientation information of the first magnetic field sensor 103P and the spacing information.

This may include the steps as follows:

Step S120: Navigate the learning catheter through each sub-region 1011 of the target region 101 (e.g., a cardiac chamber).

Step S130: In this process, continuously and simultaneously collect the following data:

1. Magnetic field strength information of the first magnetic field sensor 103P on the learning catheter. This magnetic field strength information will be provided to the controller 40 and used to derive spatial position and orientation information of the first magnetic field sensor 103P at various instants of time.

2. Relative voltage information of the first electrode 104S on the learning catheter with respect to a given reference position. The reference position may be generally selected as a relatively constant position, such as a body surface position, or a position within the cardiac chamber (usually the coronary sinus). The voltage information contains voltages produced in response to stimuli applied in various stimulus modes as discussed above.

3. Magnetic field strength information of second magnetic field sensors 103Q of the electrode patches 102. This magnetic field strength information will be provided to the controller 40 and used to derive spatial position information of the second magnetic field sensors 103Q (and hence of the respective electrode patches 102) at various instants of time.

Step S140: During data collection, continuously determine by the controller 40 whether sufficient data required by subsequent calculations has been gathered. If no, the learning catheter is further navigated until sufficient data has been collected. At this point, an indication may be provided on the display unit 108 to prompt the operator to end the fitting phase. The indication may be provided upon the elapse of a predetermined period of time, or when an error of continuous fitting attempts drops below a predetermined value.

Step S150: Start the positioning phase after sufficient data has been obtained. The applied catheter is inserted into the target region 101 (e.g., a cardiac chamber), and voltage information of second electrodes 104T on the applied catheter with respect to the reference position is collected.

Step S160: Calculate spatial positions of the second electrodes 104T by the controller 40 based on voltages at the second electrodes 104T at each instant of time, as described in greater detail below.

It is to be noted that, throughout the process including the fitting and positioning phases, the reference position and the electrode patches 102 for delivering stimuli must be kept relatively constant in position. Once any of the electrode patches 102 is displaced a distance exceeding a predetermined threshold for some reason during the process (e.g., turning of the patient), the fitting phase must be restarted from the beginning thereof.

As can be seen from the above description, a positioning algorithm according to the present invention relies mainly on the following principles:

1. Apply a magnetic field and a multi-axis electric field to a target object. The magnetic field is generated by the magnetic field generation unit 10, and the multi-axis electric field is created by constant-current or constant-voltage stimuli applied between electrode patches 102 attached to a body surface of the target object.

2. Divide the target region 101 into small sub-regions 1011, and for each sub-region 1011, create fitted models describing voltage-to-distance mappings by fitting data of voltages measured by a first electrode 104S (which position is known) and distances from the first electrode 104S to the electrode patches 102.

3. Based on measured voltage data at second electrodes 104T to be positioned and the fitted models for a sub-region, calculate distances from the second electrodes 104T to the plurality of electrode patches 102 and hence spatial positions of the second electrodes 104T.

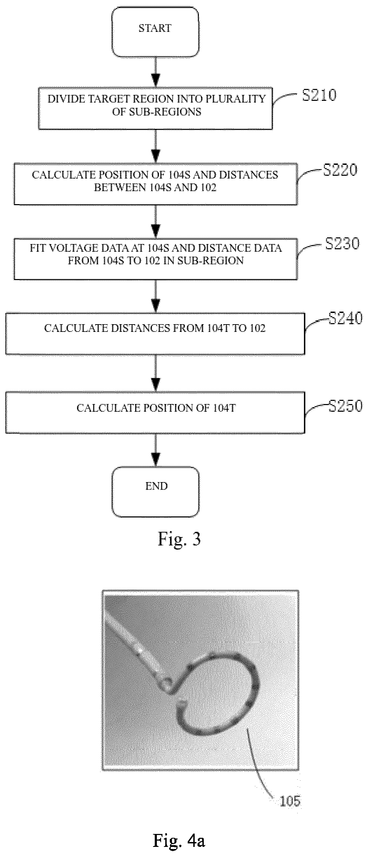

Reference is now made to Fig. 3, which schematically illustrates a specific example of the positioning algorithm.

In Step S210, a target region 101 (e.g., a cardiac chamber) is divided into a plurality of sub-regions 1011.

In Step S220, a learning catheter provided thereon with a first magnetic field sensor 103P and a first electrode 104S is advanced into the target region 101 (e.g., a cardiac chamber) and then successively navigated through each sub-region 1011. At each instant of time, magnetic field strength data is collected from the first magnetic field sensor 103P and second magnetic field sensors 103Q, and voltage data from the first electrode 104S. A position and orientation of the first magnetic field sensor 103P is calculated based on the magnetic field strength data from the first magnetic field sensor 103P, and positions of the second magnetic field sensor 103Q are calculated from the magnetic field strength data from the second magnetic field sensors 103Q. A position of the first electrode 104S is calculated based on the position and orientation of the first magnetic field sensor 103P and on a distance between the first magnetic field sensor 103P and the first electrode 104S. Based on the position of the first electrode 104S and the positions of the second magnetic field sensors 103Q (i.e., electrode patches 102), distances from the first electrode 104S to the second magnetic field sensors 103Q (i.e., to the electrode patches 102) are calculated.

In Step S230, in each sub-region 1011, for each of the electrode patches 102, a number of fitted models are obtained by fitting voltage data collected from the first electrode 104S in all stimulus modes associated with the specific electrode patch 102 and the distance between the first electrode 104S and the specific electrode patch 102. For each sub-region 1011, such fitted models describe relationships of voltages at various positions that the electrode 104 passes by produced in the various stimulus modes with respect to a reference position and the distances from the electrode 104 to the electrode patches 102 deployed on a body surface of the target object. Assuming that there are M electrode patches 102 on the body surface, each pair of which is configured to apply a stimulus, then for each electrode patch 102, there are (M-1) stimulus modes associated with the specific electrode patch 102. In these (M-1) stimulus modes, at each sampling time, (M-1) voltages are produced at the first electrode 104S, giving (M-1) voltage-distance data pairs. Accordingly, (M-1) fitted models described respective voltage-to-distance mappings can be obtained. Thus, for each sub-region 1011, there is a total of M*(M-1) fitted models for the M electrode patches. Preferably, the fitting is accomplished using a linear fitting method, more preferably, a least-square linear fitting method. It is to be noted that, as would be appreciated by those skilled in the art, in some other embodiments, other fitting methods than a linear fitting method may also be used, and the present invention is not limited in this regard.

In Step S240, for a second electrode 104T on an applied catheter, which is to be positioned, for each electrode patch 102, a distance from the second electrode 104T to the electrode patch 102 is calculated based on the fitted models for the electrode patch 102 and for a sub-region 1011, where the applied catheter is situated, and on a voltage at the second electrode 104T.

For each electrode patch 102, there are (M-1) fitted models. Preferably, a fitted model of the (M-1) fitted models for the electrode patch 102, which satisfies a first predetermined criterion, may be selected as a target fitted model, and the distance between the second electrode 104T and the electrode patch 102 may be calculated based on the target fitted model. For example, the fitted model selected from the (M-1) fitted model may be the one with the least error of fitting. Of course, in some other embodiments, multiple fitted models may be selected for weighting calculation.

In Step S250, a position of the second electrodes 104T to be positioned is calculated based on the distances between the second electrode 104T to the electrode patches 102. The position of the second electrode 104T may be calculated based on the distances between the second electrode 104T and each of the electrode patches 102. That is, the calculation may involve all the electrode patches 102. Alternatively, at least three electrode patches 102 may be selected from the electrode patches 102, which satisfy a second predetermined criterion, as target electrode patches, and the position of the second electrode 104T may be calculated based on the distances between the second electrode 104T and the target electrode patches. For example, the at least three electrode patches 102 involved in the calculation may be selected as those with good data consistency and good attachment.

The learning catheter with the first magnetic field sensor 103P and the first electrode 104S, after being advanced into a cardiac chamber, may be navigated therein for a period of time. At same sampling time, magnetic field strength data and voltage data may be simultaneously collected from the first magnetic field sensor 103P and the first electrode 104S, respectively. A position and orientation of the first magnetic field sensor 103P may be calculated from the magnetic field strength data from the first magnetic field sensor 103P, and a position of the first electrode 104S may be calculated based on the position and orientation of the first magnetic field sensor 103P and a distance between the first magnetic field sensor 103P and the first electrode 104S. Specifically, the spatial position of the first electrode 104S (second position) may be calculated according to the equation $P_2 = P_1 + D_1 \cdot d'$, where $P_2$ is the spatial position of the first electrode 104S (second position), $P_1$ and $D_1$ are the spatial position and orientation (from the first magnetic field sensor 103P toward the first electrode 104S) of the first magnetic field sensor 103P (first position), respectively, and $d'$ is the spatial distance between the first magnetic field sensor 103P and the first electrode 104S.

In an exemplary embodiment, a fourth position and a fifth position may be defined on the applied catheter. Moreover, in the positioning phase, the data collection unit 30 may further simultaneously collect, at each sampling time, magnetic field strength information of the fourth position and voltage information of the fifth position with respect to the reference position in all stimulus modes. Accordingly, the controller 40 may calculate spatial position information of the fifth position based on spatial position and orientation information of the fourth position and on a spatial distance between the fourth and fifth positions, and may calculate distance information between the fifth position and an electrode patch based on the spatial position information of the fifth position and on spatial position information of the electrode patch. The voltage information of the fifth position with respect to the reference position in different stimulus modes and the distance information between the fifth position and the electrode patch may be obtained in the form of voltage-distance data pairs of a second type, which may be used to update the fitted models for a corresponding sub-region. It is to be noted that, as would be appreciated by those skilled in the art, in the positioning phase, the controller can determine, at each sampling time, the sub-region where the applied instrument is currently situated, based on the spatial position and orientation information of the fourth position.

For example, the learning and applied catheters may be provided by the same single catheter. In this case, the fourth position may coincide with the aforementioned first position, and the fifth position may coincide with the aforementioned second position. The controller 40 can continuously acquire new voltage-distance data pairs (of the second type) in the positioning phase, which can also be used in the fitting calculations. Alternatively, the applied and learning catheters may be separate catheters. In this case, another first magnetic field sensor 103P may be provided at the fourth position of the applied catheter, and another first electrode 104S at the fifth position. In this way, the controller 40 can similarly continuously obtain new voltage-distance data pairs (of the second type) in the positioning phase.

The continuously obtained voltage-distance data pairs of the second type may be handled in any of the following ways, with the third one being preferred:

a. Doing nothing to the fitted models derived in the fitting phase. That is, further fitting calculations are not performed with the voltage-distance data pairs of the second type, in order to avoid increasing computational overhead.

b. Using the voltage-distance data pairs of the second type to update the fitted models for the corresponding sub-region 1011 derived from voltage-distance data pairs of the first type, thereby obtaining new fitted models. In this way, both the voltage-distance data pairs of the first type for the sub-region 1011 previously accumulated in the fitting phase and the voltage-distance data pairs of the second type for the same sub-region 1011 continuously obtained in the positioning phase are used in fitting calculations. Thus, the fitted models can be continuously updated with new data pairs, allowing subsequent positioning calculations to be conducted with the newest fitted models.

c. For each sub-region 1011, using only voltage-distance data pairs of the first and/or second types accumulated over a previous period of time in fitting calculations. That is, data used in the fitting calculations is fed from a queue, similarly to a first in, first out (FIFO) mechanism. This is because, considering that the data collection environment would inevitably change slowly over time, conducting the fitting calculations only with data obtained over a previous period of time can avoid accuracy of the fitted models from deteriorating due to the use of earlier historical data. In this way, the fitted models can also be continuously updated with new data pairs, and the subsequent positioning calculations are also allowed to be carried out with the newest fitted models.

It would be appreciated that, in the voltage-distance data pairs used to derive the fitted models in the above embodiments, the voltage data is obtained from direct measurements of the first electrode(s) 104S, and the distance data is spatial distance information between the first electrode 104S and electrode patches 102. Additionally, the position information of the first electrode 104S is calculated based on information about a position of the first magnetic field sensor 103P and a physical spacing between the first magnetic field sensor 103P and the first electrode 104S. Therefore, it is considered that the fitted models derived from these two types of data can reflect a true correlation between them. This is stressed because, apart from the trustable position data of the first electrode 104S that is spaced from the first magnetic field sensor 103P at a known distance, once the position of any second electrode 104T to be positioned is completed, similar voltage-distance data pairs can also be obtained from its data. However, as the position information of this second electrode 104T is calculated according to the positioning method of the present invention, it may be associated with some errors per se.

In another embodiment, the controller 40 may collect voltage information at a third position with respect to the reference position in different stimulus modes and distance information between the third position and the electrode patches 102 in the form of voltage-distance data pairs of a third type, screen these voltage-distance data pairs for valid ones, and update the fitted models for a corresponding sub-region 1011 using the valid data pairs. For example, new voltage-distance data pairs (i.e., voltage-distance data pairs of the third type) may be further obtained from a positioning result for a second electrode 104T obtained based on the fitted models and may be subjected to a screening process prescribed with certain criteria (e.g., each voltage-distance data pair of the second electrode 104T may be used in fitting calculations for the corresponding sub-region 1011, and if a reduction in error of fitting is obtained, it may be considered as "valid" and used; otherwise, it may be considered as "invalid" and discarded). In this way, all the valid data pairs can also be used in updating the fitted models.

Thus, with the positioning system of the present invention, based on information about a limited number of positions (e.g., positions that a learning catheter is navigated through) in a target region 101 in need of positioning (e.g., a cardiac chamber), spatial coordinates of any position can be calculated (simply by collecting voltage measurements at the position), enabling positioning and tracking of a device to be positioned (e.g., a catheter) within the target region 101 (e.g., a cardiac chamber).

The positioning system is further explained below in the context of its use in a loop catheter, as an example. The loop catheter serves as both a learning catheter and an applied catheter.

Reference is now made to Figs. 4a to 4c. Fig. 4a is a schematic diagram showing the structure of a steerable loop catheter according to an embodiment of the present invention. Fig. 4b schematically illustrates an arrangement of sensors on the loop catheter. Fig. 4c schematically illustrates a positioning result for the loop catheter at a certain instant of time. As shown in Figs. 4a and 4b, the loop catheter includes a shaft and a loop. On the shaft of the loop catheter, there are arranged two first electrodes 104S1 and 104S2 (at second positions) and one first magnetic field sensor 103P (at a first position). The loop is provided thereon with 10 second electrodes 104T (at third positions), denoted respectively as 104T1, 104T2 and so forth. The first magnetic field sensor 103P is spaced at known fixed spatial distances from the first electrodes 104S1 and 104S2, denoted respectively as $d'_{104S1}$ and $d'_{104S2}$. The positioning system of the present invention is able to position the electrodes (the 10 second electrodes 104T on the loop) based on limited known information (the position of the first magnetic field sensor 103P and its spatial distances to the first electrodes 104S1 and 104S2).

The loop catheter is advanced into the right atrium via a vascular access, and a ten-electrode catheter is placed in the coronary sinus. One electrode on the ten-electrode catheter is taken as a reference electrode, and the ten-electrode catheter is not move any longer throughout the surgical procedure.

Similar to the embodiment shown in Fig. 1, six electrode patches 102, each provided with a second magnetic field sensor 103Q, are attached to a body surface at different locations (R, L, H, F, C and B). Every possible pair of the electrode patches 102 is used to apply a stimulus (e.g., a 1-mA constant current) along a respective stimulus axis. Thus, there is a total of 15 stimulus axes (RL, HF, CB, CR, CL, CH, CF, RB, LB, HB, FB, RF, LF, HR and HL).

In a fitting phase, the manipulating handle 106 is manipulated to navigate a tip portion of the catheter through each sub-region of the right atrium, and the following data is collected at each sampling time:

1. magnetic field strength at the first magnetic field sensor 103P;
2. magnetic field strength at the 6 second magnetic field sensors 103Q; and
3. voltage information of the 12 electrodes (the two first electrodes 104S on the shaft and the ten second electrodes 104T on the loop) with respect to the reference position in the 15 stimulus modes.

The controller 40 calculates: position coordinates and an orientation vector of the first magnetic field sensor 103P based on the magnetic field strength at the first magnetic field sensor 103P; and position coordinates of the second magnetic field sensors 103Q based on the magnetic field strength at the second magnetic field sensors 103Q. At a certain sampling time t, a spatial position and unit orientation vector of the first magnetic field sensor 103P, as shown in Fig. 4b, can be calculated from magnetic field strength information obtained by the first magnetic field sensor 103P. The spatial position of the first magnetic field sensor 103P is denoted as $P_{103}$, and its unit orientation vector is denoted as $D_{103}$ which is oriented from the first electrode 104S1 toward the first electrode 104S2.

Thus, a position $P_{104S1}$ of the first electrode 104S1 can be calculated as:

$$P_{104S1} = P_{103P} - D_{103P} \cdot d'_{104S1},$$

and a position $P_{104S2}$ of the first electrode 104S2 as:

$$P_{104S2} = P_{103P} + D_{103P} \cdot d'_{104S2}.$$

In these equations, $d'_{104S1}$ denotes the spatial distance between the first electrode 104S1 and the first magnetic field sensor 103P, and $d'_{104S2}$ represents the spatial distance between the first electrode 104S2 and the first magnetic field sensor 103P.

Further based on position information of the electrode patches 102 (collected from the respective second magnetic field sensors 103Q), distances from 104S1 and 104S2 to the 6 electrode patches 102 at the sampling time can be derived. For convenience, only 104S1 is described below as a representative example, but it would be appreciated by those skilled in the art that information from both first electrodes 104S can be used in fitting calculations.

Additionally, voltages measured at the first electrodes 104S1 and 104S2 are denoted as $U_{104S1}$ and $U_{104S2}$, respectively. Note that each of the voltages has a 15×1 vector corresponding to voltages produced in the 15 stimulus modes.

In this example, at each sampling time, for any of the electrode patches 102, there are a total of 5 stimulus modes (corresponding to the respective possible pairs that it can constitute with the other 5 electrode patches). From the distance from the first electrode 104S1 to this electrode patch 102, denoted as $d_i$, and from the 5 voltages produced at the first electrode 104S1 in the 5 stimulus modes, denoted as v1, v2, ..., v5, 5 voltage-distance data pairs can be obtained. The same applies to all the other electrode patches 102. Thus, at each sampling time, a total of 6 × 5 = 30 voltage-distance data pairs can be obtained.

Reference is now made to Fig. 5, which schematically illustrates how the target region is divided. As shown in Fig. 5, the cardiac chamber in need of positioning (target region 101) is divided into a plurality of sub-regions 1011 so as to ensure that a learning catheter can be navigated through as many sub-regions 1011 as possible. During the navigation of the learning catheter through each sub-region 1011, it moves along a travel path 111, and in this process, voltage-distance data pairs are continuously accumulated. Subjecting the voltage-distance data pairs accumulated within the sub-region 1011 to least-square linear fitting can result in 30 fitted models.

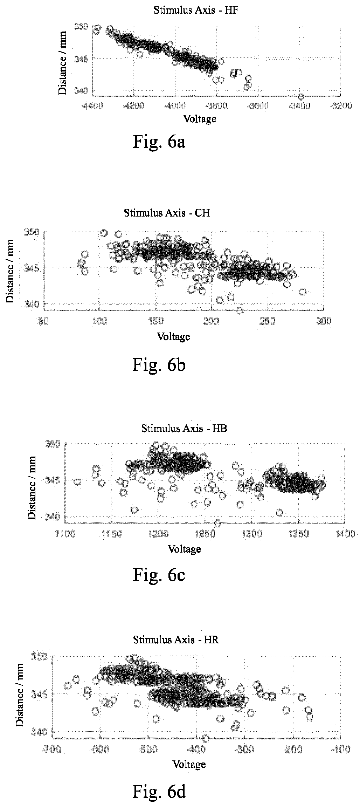

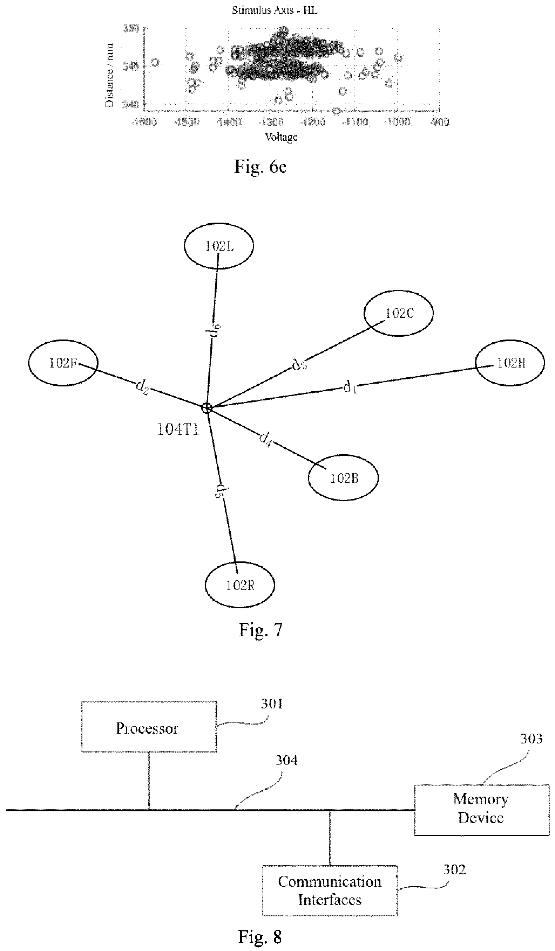

For example, the electrode patch 102H is associated with five stimulus modes: HF, CH, HB, HR and HL. Figs. 6a to 6e show schematic scatter plots of 5 sets of voltage-distance data pairs accumulated within a sub-region during a fitting phase in the respective 5 stimulus modes. In each scatter plot, the horizontal axis represents voltages collected at the first electrode 104S1 in the respective stimulus mode, and the vertical axis represents a distance between the first electrode 104S1 and the electrode patch 102H.

Results of subjecting the 5 sets of voltage-distance data pairs obtained along the respective stimulus axes shown in Figs. 6a to 6e to least-square linear fitting are shown in Table 1 below.

Table 1 Fitting Results for Stimulus Axes

| Stimulus Axis | HF | CH | HB | HR | HL |
|---|---|---|---|---|---|
| Slope | -0.010 | -0.027 | -0.017 | -0.012 | 0.004 |
| Intercept | 304.256 | 350.955 | 366.886 | 340.347 | 350.440 |
| Error of Fitting | 0.176 | 1.550 | 1.673 | 1.837 | 2.798 |

As can be seen from Table 1, among the 5 stimulus axes associated with the electrode patch 102H, the HF stimulus mode has the least error of fitting and hence the highest linearity. Accordingly, in the subsequent positioning phase, for this sub-region 1011, only the fitting results obtained along the HF axis may be used in calculating distances of the second electrodes 104T to the electrode patch 102H.

This process involving the data fitting and screening for each electrode patch 102 may be repeated in each sub-region 1011, and the results may be stored in a memory device. The fitting phase ends after this is done.

In the subsequent positioning phase, assuming that the second electrode 104T1 (see Fig. 4b) is to be positioned and that a voltage $U_{104T1}$ (also in the form of a $15 \times 1$ vector) is collected at the second electrode 104T1 at a certain sampling time, then a positioning process for the second electrode 104T1 may include:

1. determining a sub-region 1011 where the loop catheter is currently located, based on a position of the first magnetic field sensor 103P;

2. calculating distances from the second electrode 104T1 to the electrode patches 102 based on calculation results for the current sub-region 1011 obtained in the fitting phase; and

3. using triangulation to derive position information of the second electrode 104T1 from position data of the electrode patches 102 (collected from the second magnetic field sensors 103Q) and from the calculated distances from the second electrode 104T1 to the electrode patches 102.

Specifically, reference is made to Fig. 7, a schematic illustration of the distances from the second electrode 104T1 (second position) to the electrode patches 102. As shown in Fig. 7, position coordinates of the 6 electrode patches 102H, 102F, 102C, 102B, 102R and 102L can be obtained from the respective second magnetic field sensors 103Q thereon. Assuming that, at a sampling time, the position coordinates of the electrode patches 102 are obtained as:

102H: $(x_1, y_1, z_1)$
102F: $(x_2, y_2, z_2)$
102C: $(x_3, y_3, z_3)$
102B: $(x_4, y_4, z_4)$
102R: $(x_5, y_5, z_5)$
102L: $(x_6, y_6, z_6)$,

when denoting the distances from the second electrode 104T1 to the electrode patches 102 as $d_1, d_2 \cdots d_6$,
and position coordinate of the second electrode 104T1 as $(x_0, y_0, z_0)$, we can obtain the following system of equations:

$$\begin{cases} (x_0 - x_1)^2 + (y_0 - y_1)^2 + (z_0 - z_1)^2 = d_1^2 \\ (x_0 - x_2)^2 + (y_0 - y_2)^2 + (z_0 - z_2)^2 = d_2^2 \\ (x_0 - x_3)^2 + (y_0 - y_3)^2 + (z_0 - z_3)^2 = d_3^2 \\ (x_0 - x_4)^2 + (y_0 - y_4)^2 + (z_0 - z_4)^2 = d_4^2 \\ (x_0 - x_5)^2 + (y_0 - y_5)^2 + (z_0 - z_5)^2 = d_5^2 \\ (x_0 - x_6)^2 + (y_0 - y_6)^2 + (z_0 - z_6)^2 = d_6^2 \end{cases}.$$

Subtracting each of the other equations from the first equation gives:

$$\begin{cases} x_0(x_2 - x_1) + y_0(y_2 - y_1) + z_0(z_2 - z_1) = \gamma_1 \\ x_0(x_3 - x_1) + y_0(y_3 - y_1) + z_0(z_3 - z_1) = \gamma_2 \\ x_0(x_4 - x_1) + y_0(y_4 - y_1) + z_0(z_4 - z_1) = \gamma_3 \\ x_0(x_5 - x_1) + y_0(y_5 - y_1) + z_0(z_5 - z_1) = \gamma_4 \\ x_0(x_6 - x_1) + y_0(y_6 - y_1) + z_0(z_6 - z_1) = \gamma_5 \end{cases},$$

where

$$\begin{cases} \gamma_1 = \dfrac{d_1^2 - d_2^2 + x_2^2 + y_2^2 + z_2^2 - x_1^2 - y_1^2 - z_1^2}{2} \\[2ex] \gamma_2 = \dfrac{d_1^2 - d_3^2 + x_3^2 + y_3^2 + z_3^2 - x_1^2 - y_1^2 - z_1^2}{2} \\[2ex] \gamma_3 = \dfrac{d_1^2 - d_4^2 + x_4^2 + y_4^2 + z_4^2 - x_1^2 - y_1^2 - z_1^2}{2} \\[2ex] \gamma_4 = \dfrac{d_1^2 - d_5^2 + x_5^2 + y_5^2 + z_5^2 - x_1^2 - y_1^2 - z_1^2}{2} \\[2ex] \gamma_5 = \dfrac{d_1^2 - d_6^2 + x_6^2 + y_6^2 + z_6^2 - x_1^2 - y_1^2 - z_1^2}{2} \end{cases}.$$

In the above system of equations, there are only three unknowns: $x_0$, $y_0$ and $z_0$, i.e., the coordinates of the second electrode 104T1 at the sampling time, which can be obtained simply by solving the system of equations.

Positions of the other second electrodes 104T can be calculated in a similar way to that described above for the second electrode 104T1 and, therefore, need not be described in further detail herein. The loop catheter in the cardiac chamber can be located by positioning all the second electrodes 104T. Fig. 4c shows a graphic representation of the loop catheter displayed on the display unit 108.

It is to be noted that the catheter depicted in Figs. 4a to 4c is merely intended for clear illustration. Indeed, the present invention is not limited to use with the catheter configuration shown in Figs. 4a to 4c and can be used with other catheter configurations, such as balloon and basket catheters. It is to be also noted that although least-square linear fitting has been described as being used in the above examples, use of any fitting method is contemplated in the present invention, which involves: first establishing fitted models correlating voltage data (produced in response to a stimulus applied in a stimulus mode and measured by a first electrode 104S) to distance data from the first electrode 104S to electrode patches 102 that apply the stimulus; then calculating distances from a second electrode 104T to be located to at least three electrode patches 102 based on the fitted models; and subsequently based on this distance information, calculating a position of the second electrode 104T.

Further, although the present invention has been described in most of the embodiments disclosed herein as being used to position an electrode in a cardiac chamber, as would be appreciated by those skilled in the art, it can also be used in other surgical applications requiring the use of an interventional or implantable device for invasive diagnosis or treatment, such as neurosurgery, tumor ablation and pulmonary angioplasty.

It would be appreciated that, in the field of three-dimensional electrophysiological mapping, high-density mapping analysis is being more and more recognized as an important trend of development. For high-density mapping, accurate catheter positioning is indispensable. Among existing catheter positioning techniques, magnetic field-based ones are expensive despite their higher accuracy. Ordinary electrodes used in electric field-based positioning techniques can acquire data required for positioning during a mapping process, resulting in significant cost savings. However, due to strong non-linear distortion of an electric field in the human body, it is difficult for such techniques to provide guaranteed accuracy. For these reasons, most commercially available products employ both magnetic and electric field-based techniques, in order to be provided at controlled cost while offering satisfactory positioning accuracy. Based on the fact that an electric field is nearly linear locally in some regions, the present invention divides a region in need of position (i.e., a target region) into a plurality of sub-regions and then establishes a plurality of fitted models for each sub-region for positioning. Since most calculations involved in the present invention are linear, not only a small computational overhead can be achieved, but operation of a learning catheter in an early stage can be completed in a very short time.

Based on the same inventive concept, the present invention further provides an interventional surgical system including the positioning system and the interventional device discussed above. The interventional device may be a loop catheter, a balloon catheter, a basket catheter or the like.

The present invention further provides a positioning method for an interventional device, which corresponds to the positioning system discussed above. The interventional device includes a learning instrument and an applied instrument, which are configured to be placed in a target region of a target object. The positioning method includes:

dividing the target region into a plurality of sub-regions;

in a fitting phase, obtaining magnetic field strength information at a first position of the learning instrument, magnetic field strength information at at least three electrode patches disposed on a surface of the target object and voltage information at a second position on the learning instrument with respect to a reference position in all stimulus modes, which are simultaneously collected in each of the sub-regions, calculating spatial position information of the second position based on the magnetic field strength information at the first position and on spatial distance information between the first and second positions, and deriving fitted models describing voltage-to-distance mappings in each of the sub-regions from voltage information at the second position with respect to the reference position at different stimulus modes and from distance information between the second position and the electrode patches; and

in a positioning phase, obtaining voltage information simultaneously collected in different stimulus modes at a third position on the applied instrument with respect to the reference position, information about a sub-region where the applied instrument is situated and spatial position information of the electrode patches and calculating spatial position information of the third position using the fitted models for the sub-region and spatial position information of the electrode patches, wherein the spatial position information of the electrode patches is calculated from magnetic field strength information at the electrode patches.

It is to be noted that, as would be appreciated by those skilled in the art, spatial position and orientation information of the first position may be first calculated from the magnetic field strength information at the first position, and the spatial position information of the second position may be then derived from the spatial position and orientation information of the first position and the spatial distance information between the first and second positions. Moreover, the distance information between the second position and the electrode patches may be calculated from the spatial position information of the second position and the spatial position information of the electrode patches.

In an exemplary embodiment, deriving the fitted models describing the voltage-to-distance mappings in each of the sub-regions from the voltage information at the second position with respect to the reference position at the different stimulus modes and from the distance information between the second position and the electrode patches may include: gathering the voltage information at the second position with respect to the reference position in the different stimulus modes and the distance information between the second position and the electrode patches into voltage-distance data pairs of a first type and deriving the fitted models describing the voltage-to-distance mappings in the different stimulus modes in each of the sub-regions by fitting the voltage-distance data pairs.

In an exemplary embodiment, deriving the fitted models describing the voltage-to-distance mappings in each of the sub-regions from the voltage information at the second position with respect to the reference position at the different stimulus modes and from the distance information between the second position and the electrode patches may include: for each of the electrode patches, gathering voltage information at the second position with respect to the reference position in different stimulus modes associated with the electrode patch and distance information between the second position and the electrode patch into voltage-distance data pairs of a first type and derive fitted models in the different stimulus modes associated with the electrode patch in each of the sub-regions.

In an exemplary embodiment, calculating the spatial position information of the third position using the fitted models for the sub-region and the spatial position information of the electrode patches may include: calculating distance information between the third position and the electrode patches using the fitted models for the electrode patches in the sub-region; and calculating the spatial position information of the third position based on the distance information between the third position and the electrode patches and spatial position information of the electrode patches.

Further, calculating the distance information between the third position and the electrode patches using the fitted models for the electrode patches in the sub-region may include:

selecting a fitted model satisfying a first predetermined criterion from the fitted models for the different stimulus modes associated with the electrode patch as a target fitted model; and
calculating the distance information between the third position and the electrode patch using the target fitted model.

In an exemplary embodiment, calculating the spatial position information of the third position using the fitted models for the sub-region and the spatial position information of the electrode patches may include:

> selecting at least three electrode patches satisfying a second predetermined criterion from the electrode patches as target electrode patches; and
> calculating distance information between the third position and the target electrode patches using the fitted models for the sub-region and calculating the spatial position information of the third position based on spatial position information of the target electrode patches.

Further, calculating the distance information between the third position and the target electrode patches using the fitted models for the sub-region may include:
calculating the distance information between the third position and the target electrode patches using fitted models for the target electrode patches in the sub-region.

Furthermore, calculating the distance information between the third position and the target electrode patches using the fitted models for the target electrode patches in the sub-region may include:
calculating the distance information between the third position and the target electrode patches using target fitted models for the target electrode patches in the sub-region.

Optionally, the applied instrument may further include a fourth position, wherein the positioning method further includes:
in the positioning phase, collecting magnetic field strength information at the fourth position, calculating spatial position and orientation information of the fourth position based on the magnetic field strength information at the fourth position and deriving information about a sub-region wherein the applied instrument is situated from the spatial position and orientation information of the fourth position.

Optionally, the applied instrument may further include a fifth position, wherein the positioning method further includes:
in the positioning phase, simultaneously collecting voltage information at the fifth position with respect to the reference position in all the stimulus modes, calculating spatial position information of the fifth position based on spatial position and orientation information of the fourth position and spatial distance information between the fourth and fifth positions, calculating distance information between the fifth position and the electrode patches based on the spatial position information of the fifth position and spatial position information of the electrode patches, gathering the voltage information at the fifth position with respect to the reference position in the different stimulus modes and the distance information between the fifth position and the electrode patches into voltage-distance data pairs of a second type, and updating the fitted models for the sub-region using the voltage-distance data pairs of the second type.

Optionally, the positioning method may further include:
gathering voltage information at the third position with respect to the reference position in different stimulus modes and distance information between the third position and the electrode patches into voltage-distance data pairs of a third type; screening out valid data pairs from the voltage-distance data pairs of the third type; and updating the fitted models for the sub-region using the valid data pairs.

Based on the same inventive concept, the present invention further provides an electronic device. Fig. 8 is a schematic block diagram of the electronic device according to an embodiment of the present invention. As shown in Fig. 8, the electronic device includes a processor 301 and a memory device 303 storing therein a computer program which, when executed by the processor 301, implements the positioning method as defined above.

As shown in Fig. 8, the electronic device may further include communication interfaces 302 and communication buses 304. Communication between the processor 301, the communication interfaces 302 and the memory device 303 may be accomplished through the communication buses 304. Each of the communication buses 304 may be, for example, a peripheral component interconnect (PCI) bus or an extended industry standard architecture (EISA) bus. The communication buses 304 may include address buses, data buses, control buses, etc. Although they are represented by only one bold line in the figure for ease of illustration, this does not mean that there is only one bus, or only one type of bus. The communication interfaces 302 are configured to enable communication of the electronic device with other devices.

The processor 301 suitable for use in this invention may be a central processing unit (CPU) or other general-purpose processor, a digital signal processor (DSP), an application specific integrated circuit (ASIC), a field-programmable gate array (FPGA) or other programmable logic device, a discrete gate or transistor-transistor logic device, a discrete hardware component, or the like. The general-purpose processor may be a microprocessor or any other conventional processor. The processor 301 may serve as a control center of the electronic device, and the various components in the electronic device may be interconnected with various interfaces and lines.

The memory device 303 can be used to store the computer program. The processor 301 implements various functions of the electronic device by running or executing the computer program stored in the memory device 303 and calling the data stored in the memory device 303.

The memory device 303 may include non-volatile and/or volatile memory. Non-volatile memory may include read-only memory (ROM), programmable ROM (PROM), electrically programmable ROM (EPROM), electrically erasable programmable ROM (EEPROM) or flash memory. Volatile memory may include random-access memory (RAM) or external cache memory. For illustration rather than limitation, RAM is available in many forms such as static RAM (SRAM), dynamic RAM (DRAM), synchronous DRAM (SDRAM), double-data-rate SDRAM (DDRSDRAM), enhanced SDRAM (ESDRAM), Synchlink DRAM (SLDRAM), Rambus direct RAM (RDRAM), Direct Rambus DRAM (DRDRAM), Rambus DRAM(RDRAM), etc.

The present invention further provides a readable storage medium storing therein a computer program which, when executed by a processor, implements the positioning method as defined above.

According to embodiments of the present invention, the readable storage medium may be implemented as one of various computer-readable media, or any combination thereof. Each of the readable media may be a computer-readable signal medium or a computer-readable storage medium. The computer-readable storage medium may be, for example, but is not limited to, an electronic, magnetic, optical, electromagnetic, infrared, or semiconductor system, apparatus, or device, or any suitable combination of the foregoing. More specific examples (a non-exhaustive list) of the computer-readable storage medium would include the following: an electrical connection having one or more wires, a portable computer diskette, a hard disk, a random access memory (RAM), a read-only memory (ROM), an erasable programmable read-only memory (EPROM or Flash memory), an optical fiber, a portable compact disc read-only memory (CD-ROM), an optical storage device, a magnetic storage device, or any suitable combination of the foregoing. As used herein, the phase "computer-readable storage medium" may refer to any tangible medium that can contain, or store a program for use by or in connection with an instruction execution system, apparatus, or device.

The computer-readable signal medium may include a propagated data signal with computer-readable program code embodied therein, for example, in baseband or as part of a carrier wave. Such a propagated signal may take any of a variety of forms, including, but not limited to, electro-magnetic, optical, or any suitable combination thereof. The computer-readable signal medium may also be any computer-readable medium that is not a computer-readable storage medium and that can communicate, propagate, or transport a program for use by or in connection with an instruction execution system, apparatus, or device.

In summary, compared with the prior art, the positioning system and positioning method for interventional device, interventional surgical system, electronic device and storage medium have the advantages as follows: a target region is divided into a plurality of sub-regions, and in a fitting phase, fitted models describing voltage-to-distance mappings in each sub-region are established with a learning instrument. In a subsequent positioning phase, distances between a third position on an applied instrument and electrode patches are determined based on the fitted models for a sub-region where the applied instrument is situated, and a location of the third position is determined based on the distances between the third position and the electrode patches and spatial position information of the electrode patches. In this way, the applied instrument can be positioned, and position, orientation and shape information of the applied instrument in the target region of the target object can be determined. According to the present invention, the target region is divided into the sub-regions so that each sub-region is small enough to allow an electric field to be distributed linearly therein and thus to allow the fitted models to be established for each sub-region. Using such fitted models, the distances between the third position and the electrode patches can be more accurately determined, achieving more accurate positioning calculations. Further, since fitted models are established for each sub-region according to the present invention, computational burden in the fitting calculations is small. Thus, effective reductions in computational burden and time savings can be achieved.

Notably, computer program code for carrying out operations of the present invention may be written in any combination of one or more programming languages, including an object-oriented programming language such as Java, Smalltalk, C++ or the like and conventional procedural programming languages, such as the "C" programming language or similar programming languages. The program code may execute entirely on the user's computer, partly on the user's computer, as a stand-alone software package, partly on the user's computer and partly on a remote computer or entirely on a remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

It is to be noted that the devices and methods disclosed in the embodiments herein may also be implemented in other ways and the device embodiments described above are merely illustrative. For example, the flowcharts and block diagrams in the figures illustrate the architecture, functionality and operation of possible implementations of devices, methods, and computer program products according to various embodiments of the present invention. In this regard, each block in the flowcharts or block diagrams may represent a module, segment, or portion of instructions, which comprises one or more executable instructions for implementing the specified logical function(s). It is to be also noted that, in some alternative implementations, the functions noted in the blocks may occur out of the order noted in the figures. For example, two blocks shown in succession may, in fact, be executed substantially concurrently, or the blocks may sometimes be executed in the reverse order, depending upon the functionality involved. It is to be further noted that each block of the block diagrams and/or flowchart illustrations, and combinations of blocks in the block diagrams and/or flowchart illustrations, can be implemented by special purpose hardware-based systems that perform the specified functions or acts or carry out combinations of special purpose hardware and computer instructions.

Further, the various functional modules in the embodiments herein may be integrated into a discrete component, or provided as separate modules. Alternatively, two or more of the modules may be integrated into a discrete component.

The description presented above is merely that of a few preferred embodiments of the present invention and is not intended to limit the scope thereof in any sense. Any and all changes and modifications made by those of ordinary skill in the art based on the above teachings fall within the scope as defined in the appended claims. Apparently, those skilled in the art can make various modifications and variations to the present invention without departing from the spirit and scope thereof. Accordingly, the invention is intended to embrace all such modifications and variations if they fall within the scope of the appended claims and equivalents thereof.

**Claims**

1. A positioning system for an interventional device, wherein the interventional device comprises a learning instrument and an applied instrument, which are configured to be placed in a target region of a target object,

   wherein the positioning system comprises a magnetic field generation unit, a stimulus control unit, a data collection unit and a controller,
   the magnetic field generation unit configured to generate a magnetic field passing through the target object;
   the stimulus control unit configured to create an N-axis electric field by applying stimuli between at least three electrode patches disposed on a surface of the target object, where $N \geq 3$,
   the controller configured to divide the target region into a plurality of sub-regions;
   the data collection unit configured to: in a fitting phase, in each of the sub-regions, simultaneously collect magnetic field strength information at a first position on the learning instrument, magnetic field strength information at the electrode patches and voltage information at a second position on the learning instrument with respect to a reference position in all stimulus modes; and in a positioning phase, simultaneously collect magnetic field strength information at the electrode patches and voltage information at a third position on the applied instrument with respect to the reference position in all the stimulus modes,
   the controller further configured to: in the fitting phase, in each of the sub-regions, calculate spatial position information of the second position based on magnetic field strength information at the first position and on spatial distance information between the first and second positions and derive, for each of the sub-regions, fitted models describing voltage-to-distance mappings, from voltage information at the second position with respect to the reference position in different stimulus modes and distance information between the second position and the electrode patches; and in the positioning phase, based on voltage information at the third position with respect to the reference position in the different stimulus modes and information about a sub-region where the applied instrument is situated, calculate spatial position information of the third position using the fitted models for the sub-region and spatial position information of the electrode patches, wherein the spatial position information of the electrode patches is calculated from magnetic field strength information at the electrode patches.

2. The positioning system according to claim 1, wherein the controller is configured to gather the voltage information at the second position with respect to the reference position in the different stimulus modes and the distance information between the second position and the electrode patches into voltage-distance data pairs of a first type and derive the fitted models describing the voltage-to-distance mappings in the different stimulus modes in each of the sub-regions by fitting the voltage-distance data pairs.

3. The positioning system according to claim 1, wherein the controller is configured to calculate distance information between the third position and at least three of the electrode patches using the fitted models in the different stimulus modes in the sub-region, and calculate the spatial position information of the third position from the distance information between the third position and the at least three of the electrode patches and spatial position information of the at least three of the electrode patches.

4. The positioning system according to claim 1, wherein the controller is configured to: in the fitting phase, for each of the electrode patches, gather voltage information at the second position with respect to the reference position in different stimulus modes associated with the electrode patch and distance information between the second position and the electrode patch into voltage-distance data pairs of a first type and derive fitted models in the different stimulus modes associated with the electrode patch, in each of the sub-regions; and

in the positioning phase, calculate distance information between the third position and the electrode patches using the fitted models for the electrode patches in a sub-region where the applied instrument is situated.

5. The positioning system according to claim 4, wherein the controller is configured to select a fitted model satisfying a first predetermined criterion from the fitted models for the different stimulus modes associated with the electrode patch as a target fitted model, and calculate distance information between the third position and the electrode patch using the target fitted model.

6. The positioning system according to claim 1, wherein the controller is configured to select at least three electrode patches satisfying a second predetermined criterion from the electrode patches as target electrode patches, calculate distance information between the third position and the target electrode patches using the fitted models for a sub-region where the applied instrument is situated, and calculate spatial position information of the third position based on spatial position information of the target electrode patches.

7. The positioning system according to claim 1, wherein the applied instrument further comprises a fourth position, wherein:

the data collection unit is further configured to collect magnetic field strength information at the fourth position in the positioning phase; and
the controller is further configured to, in the positioning phase, calculate spatial position and orientation information of the fourth position based on the magnetic field strength information at the fourth position and derive information about a sub-region wherein the applied instrument is situated from the spatial position and orientation information of the fourth position.

8. The positioning system according to claim 7, wherein the applied instrument further comprises a fifth position, wherein:

the data collection unit is further configured to simultaneously collect voltage information at the fifth position with respect to the reference position in all the stimulus modes in the positioning phase; and
the controller is further configured to: in the positioning phase, calculate spatial position information of the fifth position based on spatial position and orientation information of the fourth position and spatial distance information between the fourth and fifth positions; calculate distance information between the fifth position and the electrode patches based on the spatial position information of the fifth position and spatial position information of the electrode patches; gather the voltage information at the fifth position with respect to the reference position in the different stimulus modes and the distance information between the fifth position and the electrode patches into voltage-distance data pairs of a second type; and update the fitted models for the sub-region using the voltage-distance data pairs of the second type.

9. The positioning system according to claim 8, wherein the learning and applied instruments are provided as a single instrument, wherein the fourth position coincides with the first position, and the fifth position coincides with the second position.

10. The positioning system according to claim 1, wherein the controller is further configured to: gather voltage information at the third position with respect to the reference position in different stimulus modes and distance information between the third position and the electrode patches into voltage-distance data pairs of a third type; screen out valid data pairs from the voltage-distance data pairs of the third type; and update the fitted models for the sub-region using the valid data pairs.

11. The positioning system according to claim 1, wherein the stimulus control unit is configured to apply stimuli between the at least three electrode patches and fast switch between all the stimulus modes in a cyclic and continuous manner, wherein the data collection unit collects voltage information at the second and third positions with respect to the reference position in all the stimulus modes, or

the stimulus control unit is configured to simultaneously apply stimuli, each at a different frequency, between the at least three electrode patches, wherein the data collection unit collects and filters voltage information at the second and third positions with respect to the reference position, thereby obtaining voltage information at the second and third positions with respect to the reference position in all the stimulus modes; or
the stimulus control unit is configured to simultaneously apply stimuli, each at a different frequency, between the at least three electrode patches, wherein the data collection unit collects voltage information at the second and third positions with respect to the reference position, and the controller is configured to filter the voltage information at the second and third positions with respect to the reference position collected by the data collection unit, thereby obtaining voltage information at the second and third positions with respect to the reference position in all the stimulus modes.

12. The positioning system according to claim 1, further comprising a communication control unit, which is configured to connect the controller to the magnetic field generation unit, the stimulus control unit and the data collection unit, thereby controlling communication and data transmission between the controller and the magnetic field generation unit, the stimulus control unit and the data collection unit.

13. The positioning system according to claim 1, further comprising a display unit, which is communicatively connected to the controller and configured to display a position, orientation, shape and/or travel path of the learning instrument and/or the applied instrument in the target object.

14. The positioning system according to claim 1, wherein a first magnetic field sensor is disposed at the first position, and second magnetic field sensors is disposed on the electrode patches, wherein the data collection unit collects magnetic field strength information at the first position through the first magnetic field sensor and collects magnetic field strength information at the electrode patches through the second magnetic field sensors, and

a first voltage sensor is disposed at the second position, and a second voltage sensor is disposed at the third position, wherein the data collection unit collects voltage information at the second position with respect to the reference position through the first voltage sensor and collects voltage information at the third position with respect to the reference position through the second voltage sensor.

15. An interventional surgical system, comprising the positioning system according to any one of claims 1 to 14 and the interventional device.

16. A positioning method for an interventional device, wherein the interventional device comprises a learning instrument and an applied instrument, which are configured to be placed in a target region of a target object, wherein the positioning method comprises:

dividing the target region into a plurality of sub-regions;
in a fitting phase, obtaining magnetic field strength information at a first position of the learning instrument, magnetic field strength information at at least three electrode patches disposed on a surface of the target object and voltage information at a second position on the learning instrument with respect to a reference position in all stimulus modes, which are simultaneously collected in each of the sub-regions, calculating spatial position information of the second position based on the magnetic field strength information at the first position and on spatial distance information between the first and second positions, and deriving fitted models describing voltage-to-distance mappings in each of the sub-regions from voltage information at the second position with respect to the reference position at different stimulus modes and from distance information between the second position and the electrode patches; and
in a positioning phase, obtaining voltage information simultaneously collected in different stimulus modes at a third position on the applied instrument with respect to the reference position, information about a sub-region where the applied instrument is situated and spatial position information of the electrode patches, and calculating spatial position information of the third position using the fitted models for the sub-region and spatial position information of the electrode patches, wherein the spatial position information of the electrode patches is calculated from magnetic field strength information at the electrode patches.

17. The positioning method according to claim 16, wherein deriving the fitted models describing the voltage-to-distance mappings in each of the sub-regions from the voltage information at the second position with respect to the reference position at the different stimulus modes and from the distance information between the second position and the electrode patches comprises:
gathering the voltage information at the second position with respect to the reference position in the different stimulus modes and the distance information between the second position and the electrode patches into voltage-distance data pairs of a first type and deriving the fitted models describing the voltage-to-distance mappings in the different stimulus modes in each of the sub-regions by fitting the voltage-distance data pairs of the first type.

18. The positioning method according to claim 16, wherein deriving the fitted models describing the voltage-to-distance mappings in each of the sub-regions from the voltage information at the second position with respect to the reference position at the different stimulus modes and from the distance information between the second position and the electrode patches comprises:

for each of the electrode patches, gathering voltage information at the second position with respect to the reference position in different stimulus modes associated with the electrode patch and distance information between the second position and the electrode patch into voltage-distance data pairs of a first type and deriving fitted models in the different stimulus modes associated with the electrode patch in each of the sub-regions.

19. The positioning method according to claim 18, wherein calculating the spatial position information of the third position using the fitted models for the sub-region and the spatial position information of the electrode patches comprises:

calculating distance information between the third position and the electrode patches using the fitted models for the electrode patches in the sub-region; and
calculating the spatial position information of the third position based on the distance information between the third position and the electrode patches and spatial position information of the electrode patches.

20. The positioning method according to claim 19, wherein calculating the distance information between the third position and the electrode patches using the fitted models for the electrode patches in the sub-region comprises:

selecting a fitted model satisfying a first predetermined criterion from the fitted models for the different stimulus modes associated with the electrode patch as a target fitted model; and
calculating the distance information between the third position and the electrode patch using the target fitted model.

21. The positioning method according to claim 16, wherein calculating the spatial position information of the third position using the fitted models for the sub-region and the spatial position information of the electrode patches comprises:

selecting at least three electrode patches satisfying a second predetermined criterion from the electrode patches as target electrode patches; and
calculating distance information between the third position and the target electrode patches using the fitted models for the sub-region, and calculating the spatial position information of the third position based on spatial position information of the target electrode patches.

22. The positioning method according to claim 16, wherein the applied instrument further comprises a fourth position, wherein the positioning method further comprises:
in the positioning phase, collecting magnetic field strength information at the fourth position, calculating spatial position and orientation information of the fourth position based on the magnetic field strength information at the fourth position, and deriving information about a sub-region wherein the applied instrument is situated from the spatial position and orientation information of the fourth position.

23. The positioning method according to claim 22, wherein the applied instrument further comprises a fifth position, wherein the positioning method further comprises:
in the positioning phase, simultaneously collecting voltage information at the fifth position with respect to the reference position in all the stimulus modes, calculating spatial position information of the fifth position based on spatial position and orientation information of the fourth position and spatial distance information between the fourth and fifth positions, calculating distance information between the fifth position and the electrode patches based on the spatial position information of the fifth position and spatial position information of the electrode patches, gathering the voltage information at the fifth position with respect to the reference position in the different stimulus modes and the distance information between the fifth position and the electrode patches into voltage-distance data pairs of a second type, and updating the fitted models for the sub-region using the voltage-distance data pairs of the second type.

24. The positioning method according to claim 16, further comprising:
gathering voltage information at the third position with respect to the reference position in different stimulus modes and distance information between the third position and the electrode patches into voltage-distance data pairs of a third type; screening out valid data pairs from the voltage-distance data pairs of the third type; and updating the fitted models for the sub-region using the valid data pairs.

25. An electronic device, comprising a processor and a memory device, the memory device storing therein a computer program, which, when executed by the processor, implements the positioning method according to any one of claims 16 to 24.

26. A readable storage medium, wherein the readable storage medium stores therein a computer program, which, when executed by a processor, implements the positioning method according to any one of claims 16 to 24.

Fig. 1

START

APPLY MAGNETIC FIELD AND MULTI-AXIS ELECTRIC FIELD — S110

ADVANCE LEARNING CATHETER INTO TARGET REGION AND NAVIGATE IT THROUGHOUT TARGET REGION — S120

FITTING PHASE

COLLECT MAGNETIC FIELD STRENGTH AT EACH FIRST MAGNETIC FIELD SENSOR ON LEARNING CATHETER AND VOLTAGES AT EACH FIRST ELECTRODE — S130

SUFFICIENT DATA HAS BEEN GATHERED? — S140

NO

YES

POSITIONING PHASE

ADVANCE APPLIED CATHETER INTO TARGET REGION AND COLLECT VOLTAGES AT EACH SECOND ELECTRODE — S150

CALCULATE POSITIONS OF SECOND ELECTRODES ON APPLIED CATHETER — S160

END

Fig. 2

START

DIVIDE TARGET REGION INTO PLURALITY
OF SUB-REGIONS — S210

CALCULATE POSITION OF 104S AND DISTANCES
BETWEEN 104S AND 102 — S220

FIT VOLTAGE DATA AT 104S AND DISTANCE DATA
FROM 104S TO 102 IN SUB-REGION — S230

CALCULATE DISTANCES FROM 104T TO 102 — S240

CALCULATE POSITION OF 104T — S250

END

Fig. 3

105

Fig. 4a

Fig. 4b

Fig. 4c

Fig. 5

Stimulus Axis - HF

Fig. 6a

Stimulus Axis - CH

Fig. 6b

Stimulus Axis - HB

Fig. 6c

Stimulus Axis - HR

Fig. 6d

Stimulus Axis - HL

Fig. 6e

Fig. 7

Fig. 8

<div align="center">

### INTERNATIONAL SEARCH REPORT

</div>

| | International application No. |
|---|---|
| | **PCT/CN2022/101428** |

| A. CLASSIFICATION OF SUBJECT MATTER |
|---|
| A61B 34/20(2016.01)i |
| According to International Patent Classification (IPC) or to both national classification and IPC |

| B. FIELDS SEARCHED |
|---|
| Minimum documentation searched (classification system followed by classification symbols) |
| A61B |
| Documentation searched other than minimum documentation to the extent that such documents are included in the fields searched |
| Electronic data base consulted during the international search (name of data base and, where practicable, search terms used) |
| CNABS; WPABSC; WPABS; DWPI; VEN; CNTXT; ENTXT; ENTXTC; CNKI; web of science: 导管, 定位, 导航, 位置, 传感器, 电极, 磁场, 电场, 电流, 电压, 阻抗, 激励, 映射, 拟合, 模型, 学习, 训练, 查找表, 配准, 配对, 神经网络, 最小二乘, catheter, position+, navigat+, locat+, sensor? , electrode+, electro+, +magnetic+, current, voltage, impedance, data pair, model, map+, fit+, match+, lookup, CNN, neural network, mean square |

| C. DOCUMENTS CONSIDERED TO BE RELEVANT | | |
|---|---|---|
| Category* | Citation of document, with indication, where appropriate, of the relevant passages | Relevant to claim No. |
| PX | CN 113907880 A (SHANGHAI MICROPORT EP MEDTECH CO., LTD.) 11 January 2022 (2022-01-11) description, paragraphs 84-221 | 1-26 |
| PX | CN 113907881 A (SHANGHAI MICROPORT EP MEDTECH CO., LTD.) 11 January 2022 (2022-01-11) description, paragraphs 83-221 | 1-26 |
| A | CN 109965875 A (SICHUAN JINJIANG ELECTRONIC SCIENCE AND TECHNOLOGY CO., LTD.) 05 July 2019 (2019-07-05) description, paragraphs 32-56 | 1-26 |
| A | CN 106606372 A (SICHUAN JINJIANG ELECTRONIC SCIENCE AND TECHNOLOGY CO., LTD.) 03 May 2017 (2017-05-03) entire document | 1-26 |
| A | CN 111297470 A (BIOSENSE WEBSTER (ISRAEL), LTD.) 19 June 2020 (2020-06-19) entire document | 1-26 |

| ☑ Further documents are listed in the continuation of Box C. | ☑ See patent family annex. |
|---|---|

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **19 August 2022** | **06 September 2022** |

| Name and mailing address of the ISA/CN | Authorized officer |
|---|---|
| **China National Intellectual Property Administration (ISA/CN)** **No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**

| International application No. |
| --- |
| **PCT/CN2022/101428** |

**C.    DOCUMENTS CONSIDERED TO BE RELEVANT**

| Category* | Citation of document, with indication, where appropriate, of the relevant passages | Relevant to claim No. |
| --- | --- | --- |
| A | EP 2854634 A1 (ST JUDE MEDICAL ATRIAL FIBRILLATION DIV) 08 April 2015 (2015-04-08)<br>entire document | 1-26 |
| A | WO 2020096810 A1 (ST JUDE MEDICAL INT HOLDING SARL) 14 May 2020 (2020-05-14)<br>entire document | 1-26 |
| A | US 2020138525 A1 (ST JUDE MEDICAL INT HOLDING SARL) 07 May 2020 (2020-05-07)<br>entire document | 1-26 |
| A | US 2020138334 A1 (ST JUDE MEDICAL INT HOLDING SARL) 07 May 2020 (2020-05-07)<br>entire document | 1-26 |
| A | US 2016367168 A1 (ST JUDE MEDICAL CARDIOLOGY DIV INC.) 22 December 2016 (2016-12-22)<br>entire document | 1-26 |
| A | US 2020297413 A1 (BOSTON SCI SCIMED INC.) 24 September 2020 (2020-09-24)<br>entire document | 1-26 |

Form PCT/ISA/210 (second sheet) (January 2015)

**INTERNATIONAL SEARCH REPORT**
Information on patent family members

| | | | International application No. |
|---|---|---|---|
| | | | **PCT/CN2022/101428** |

| Patent document cited in search report | | | Publication date (day/month/year) | Patent family member(s) | | | Publication date (day/month/year) |
|---|---|---|---|---|---|---|---|
| CN | 113907880 | A | 11 January 2022 | WO | 2022007815 | A1 | 13 January 2022 |
| CN | 113907881 | A | 11 January 2022 | WO | 2022007816 | A1 | 13 January 2022 |
| CN | 109965875 | A | 05 July 2019 | | None | | |
| CN | 106606372 | A | 03 May 2017 | | None | | |
| CN | 111297470 | A | 19 June 2020 | US | 2020179057 | A1 | 11 June 2020 |
| | | | | EP | 3666184 | A1 | 17 June 2020 |
| | | | | IL | 270904 | A | 30 June 2020 |
| | | | | JP | 2020093097 | A | 18 June 2020 |
| | | | | US | 11324556 | B2 | 10 May 2022 |
| EP | 2854634 | A1 | 08 April 2015 | WO | 2014028114 | A1 | 20 February 2014 |
| | | | | JP | 2015531620 | A | 05 November 2015 |
| | | | | EP | 2854634 | B1 | 27 April 2016 |
| | | | | JP | 6095779 | B2 | 15 March 2017 |
| | | | | US | 2012302869 | A1 | 29 November 2012 |
| | | | | JP | 2016105803 | A | 16 June 2016 |
| | | | | JP | 6227684 | B2 | 08 November 2017 |
| | | | | US | 10362963 | B2 | 30 July 2019 |
| WO | 2020096810 | A1 | 14 May 2020 | JP | 2022510112 | A | 26 January 2022 |
| | | | | EP | 3852623 | A1 | 28 July 2021 |
| | | | | CN | 113260306 | A | 13 August 2021 |
| US | 2020138525 | A1 | 07 May 2020 | | None | | |
| US | 2020138334 | A1 | 07 May 2020 | | None | | |
| US | 2016367168 | A1 | 22 December 2016 | CN | 107771055 | A | 06 March 2018 |
| | | | | EP | 3282995 | A1 | 21 February 2018 |
| | | | | WO | 2016205807 | A1 | 22 December 2016 |
| | | | | JP | 2018519046 | A | 19 July 2018 |
| | | | | EP | 3282995 | B1 | 24 April 2019 |
| | | | | JP | 6728244 | B2 | 22 July 2020 |
| | | | | CN | 107771055 | B | 26 February 2021 |
| US | 2020297413 | A1 | 24 September 2020 | EP | 3941347 | A1 | 26 January 2022 |
| | | | | WO | 2020197935 | A1 | 01 October 2020 |
| | | | | CN | 113891678 | A | 04 January 2022 |

Form PCT/ISA/210 (patent family annex) (January 2015)